# EUROPEAN PATENT SPECIFICATION

(11) **EP 3 519 570 B1**
(45) Date of publication and mention of the grant of the patent: **08.06.2022**
(21) Application number: 17780673.4
(22) Date of filing: 27.09.2017
(51) Int. Cl.: C12N 15/10, A61K 47/51

(54) **METHOD TO ANALYZE AND OPTIMIZE GENE EDITING MODULES AND DELIVERY APPROACHES**
VERFAHREN ZUR ANALYSE UND OPTIMIERUNG VON GENMANIPULATIONSMODULEN UND TRANSFERANSÄTZEN
PROCÉDÉ D'ANALYSE ET D'OPTIMISATION DE MODULES D'ÉDITION GÉNIQUE ET APPROCHES DE DÉLIVRANCE

(30) Priority: 29.09.2016 EP 16191511; 16.02.2017 EP 17156440
(43) Date of publication of application: 07.08.2019
(73) Proprietor: F. Hoffmann-La Roche AG, 4070 Basel (CH)
(72) Inventor: BRINKMANN, Ulrich, 82362 Weilheim (DE); KILLIAN, Tobias, 79589 Binzen (DE)
(74) Representative: Skolaut, Alexander
(86) International application number: PCT/EP2017/074480
(87) International publication number: WO 2018/060238

(56) References cited:
- WO-A1-2007/143858
- WO-A2-2016/109840
- US-A1- 2016 058 889
- US-A1- 2016 273 001
- JAN E CARETTE ET AL: "Haploid Genetic Screens in Human Cells Identify Host Factors Used by Pathogens", SCIENCE, AMERICAN ASSOCIATION FOR THE ADVANCEMENT OF SCIENCE, vol. 326, no. 5957, 27 November 2009 (2009-11-27), pages 1231-1235, XP008150319, ISSN: 0036-8075, DOI: 10.1126/SCIENCE.1178955
- SEBASTIAN STAHL ET AL: "Loss of diphthamide pre-activates NF-[kappa]B and death receptor pathways and renders MCF7 cells hypersensitive to tumor necrosis factor (Includes Supporting Information)", PROCEEDINGS OF THE NATIONAL ACADEMY OF SCIENCES, vol. 112, no. 34, 10 August 2015 (2015-08-10), pages 10732-10737+6p, XP055245674, US ISSN: 0027-8424, DOI: 10.1073/pnas.1512863112
- GABRIELE PICCO ET AL: "A diphtheria toxin resistance marker for in vitro and in vivo selection of stably transduced human cells", SCIENTIFIC REPORTS, vol. 5, 14721, 30 September 2015 (2015-09-30), pages 1-11, XP055245105, DOI: 10.1038/srep14721
- GUPTA PRADEEP K ET AL: "The diphthamide modification on elongation factor-2 renders mammalian cells resistant to ricin", August 2008 (2008-08), CELLULAR MICROBIOLOGY, VOL. 10, NR. 8, PAGE(S) 1687-1694, XP002766811, ISSN: 1462-5814 DOI: 10.1111/j.1462-5822.2008.01159.x., the whole document
- VINCENT ROY ET AL: "A Dominant-Negative Approach That Prevents Diphthamide Formation Confers Resistance to Pseudomonas Exotoxin A and Diphtheria Toxin", PLOS ONE, vol. 5, no. 12, E15753, 23 December 2010 (2010-12-23), pages 1-7, XP055245675, DOI: 10.1371/journal.pone.0015753
- KILLIAN TOBIAS ET AL: "Disruption of diphthamide synthesis genes and resulting toxin resistance as a robust technology for quantifying and optimizing CRISPR/Cas9-mediated gene editing.", 13 November 2017 (2017-11-13), SCIENTIFIC REPORTS 13 NOV 2017, VOL. 7, NR. 1, PAGE(S) 15480, XP002775834, ISSN: 2045-2322 the whole document

## Description

The current invention is in the field of gene editing for the generation of modified cell lines or organisms. More precisely, herein is reported a method for the determination of genomic integration events.

### Background of the Invention

Technologies to engineer biological systems and organism are essential for basic science, medicine and biotechnology (Ran, et al. 2013). In the last years, several genome editing technologies have arisen, including zinc-finger nucleases (ZFNs) (Miller, et al. 2007; Sander, et al. 2011; Wood, et al. 2011), transcription activator-like effector nucleases (TALENs) (Hockemeyer, et al. 2011; Sanjana, et al. 2012; Wood, et al. 2011; Zhang, et al. 2011) and the RNA-guided CRISPR/Cas9 nuclease system (Cho, et al. 2013; Cong, et al. 2013; Makarova, et al. 2011; Ran, et al. 2013). The CRISPR/Cas9 nuclease system is guided by small RNAs containing 20 nucleotides that are complementary to a target DNA sequence (Fu, et al. 2014). In contrast to TALENs and ZFNs, the CRISPR/Cas9 system is said to be easier to design, efficient and well-suited for high-throughput and multiplexed gene editing for a variety of cell types and organism (Ran, et al. 2013). Furthermore, Gene editing technologies are essential for genetic motived tools such as in cell lines, in primary cells (somatic and pluripotent stem cells) and in fertilized oocytes for the generation of transgenic animals (drosophila melanogaster, zebrafish, mice, rats and rabbits) (McMahon, et al. 2012; Urnov, et al. 2010). The first application of therapeutic genome editing was CCR5 in autologous CD4 T-cells of HIV patients which was entered in clinic (Gori, et al. 2015; Tebas, et al. 2014). Beyond this application, gene editing has been successfully applied in a variety number of diseases at preclinical level as well as in a phase 1 clinical trial (Cox, et al. 2015; Holt, et al. 2010; Li, et al. 2011; Perez, et al. 2008; Tebas, et al. 2014; Yin, et al. 2014). The mechanism to achieve a genome editing based therapy are correction or inactivation of deleterious mutations, introduction of protective mutations, addition of therapeutic transgenes and disruption of viral DNA (Cox, et al. 2015). Hence, gene editing approaches - including those using CRISPR/Cas9 technologies might give rise to an entire new class of therapeutics for different diseases.

For therapeutic applications (as well as for effective usage in R&D), characterizations and comparisons of gene editing technologies & modules is essential. This comprises analyses and comparisons of their efficiency and specificity, as well as optimization of delivery to target cells. Optimization of delivery and assessment of specificity are critical for the safe and effective clinical translation of gene editing technologies (Gori, et al. 2015). For example, it is highly desired to reduce the off-target activity and increase specificity in CRISPR/Cas9 systems (Zhang, et al. 2015). Off-target mutations may occur frequently and at much higher rates than intended targeted mutations and insertions. This may induce genomic instability and a disruptor of functionality of otherwise normal genes (Cho, et al. 2014; Fu, et al. 2013; Mali, et al. 2013a; Pattanayak, et al. 2013; Zhang, et al. 2015). The goal is to optimize various components of the CRISPR/Cas9 system which facilitate the reduction of off-target activity without losing on-target cleavage efficiency (Zhang, et al. 2015).

One prerequisite for the development and optimization of CRISPR/Cas9 derived applications is the reliable and robust detection and exact determination of heterozygous and homozygous gene inactivation as well as non-specific and targeted integration events. Existing methods such as determination of phenotypes caused by insertions (e.g. drug resistances) or lack of phenotypes (gene inactivation) or genetic analyses / sequencing approaches do frequently not differentiate between homozygous and heterozygous inactivation. Also, existing technologies rarely address genetic composition of individual cells, or are not based on large numbers of individual gene-edited cells to support robust statistical analyses.

Picco, G., et al., disclosed diphtheria toxin resistance marker for in vitro and in vivo selection of stably transduced human cells (Scientif. Rep. 5 (2015) 1-11). Stahl, S., et al., disclosed loss of diphthamide pre-activates NF-[kappa]B and death receptor pathways and renders MCF7 cells hypersensitive to tumor necrosis factor (Includes Supporting Information) (Proc. Natl. Acad. Sci. USA 112 (2015) 10732-10737+6p).

US 2016/058889 disclosed CRISPR/Cas9-mediated gene editing (Myo-editing) is effective at correcting the dystrophin gene mutation in the mdx mice, a model for Duchenne muscular dystrophy (DMD). WO 2016/109840 disclosed cell lines for high efficiency genome editing using cas/CRISPR systems, methods of generating such cells lines, and methods of generating mutations in the genome of an organism using such cell lines.Pradeep, G.K., et al., disclosed that the diphthamide modification on elongation factor-2 renders mammalian cells resistant to ricin (Cell. Microbiol. 10 (2008) 1687-1694).

WO 2007/143858 disclosed Dph2 gene deletion mutant and uses thereof.

Carette, J.E., et al., disclosed that haploid genetic screens in human cells identify host factors used by pathogens (Science 326 (2009) 1231-1235). Roy, V., et al. disclosed a dominant-negative approach that prevents diphthamide formation confers resistance to pseudomonas exotoxin A and diphtheria toxin (PLOS ONE 5 (2010) 1-7).

### Summary of the Invention

Herein is reported a robust and simple method to quantify and optimize gene editing approaches, based on a combination of gene inactivation combined with toxin and optionally antibiotic selection. The method not only determines gene editing efficiency on a large numbers of individual cells, i.e. is suitable for a high throughput approach or use, but allows differentiating between homozygous and heterozygous integration events as well as between site specific and non-specific gene disruption and/or integration events. The simplicity and robustness of the method enables the analysis and direct comparison of efficiency and specificity of different gene editing modules, in order to identify suitable cell clones as well as integration events resulting in the desired properties for applications in research and development as well as therapy.

Herein is reported amongst other things a robust method for the quantification of CRISPR/Cas mediated gene alteration. With the method it is possible, for example, to determine efficiency of gene editing events, differences between site specific and non-(site-)specific gene disruption, and sequence integration events. Thus, the method as reported herein can be used for the quantification and/or optimization of CRISR/Cas mediated gene inactivation and integration events.

One aspect as reported herein is a method for determining the introduction of a nucleic acid into the genome of a mammalian cell, whereby the mammalian cell comprises, in one embodiment one or more, in another embodiment two, transcriptionally active alleles of a DPH1, DPH2, DPH4 and/or DPH5 gene, comprising the steps of
- transfecting the mammalian cell with one or more plasmids comprising the nucleic acid to be introduced, and the elements required for gene editing of said DPH gene,
- cultivating the transfected cell in the presence of a DPH gene transcription sensitive toxin,
- determining the introduction of a nucleic acid into the genome of the mammalian cell if the transfected cell is viable in the presence of the toxin.

In one embodiment of the methods as reported herein the DPH gene transcription sensitive toxin is selected from the group consisting of pseudomonas exotoxin, diphtheria toxin and cholix toxin.

In one embodiment of the methods as reported herein the method comprises the following steps:
- transfecting the mammalian cell with one or more plasmids comprising the nucleic acid to be introduced, a nucleic acid conferring resistance to a selection marker, and the elements required for gene editing of said DPH gene,
- cultivating the transfected cell in the absence of selection pressure,
- splitting the culture into at least two aliquots, or taking at least two samples from the culture, and
- cultivating the first aliquot or sample in the presence of a DPH gene transcription sensitive toxin, and cultivating the second aliquot or sample in the presence of the corresponding selection marker.

The method as reported herein can be used to determine gene editing efficiency. In order to do this analysis on a statistical basis a large numbers of individual cells have to be processed and analyzed individually.

In one embodiment of the methods as reported herein the mammalian cell is a multitude of mammalian cells and the method comprises directly before the step of cultivating the cell in the presence of the toxin and/or the selectable marker the step of
- depositing the cells of the transfected multitude of cells as single cells.

In one embodiment of the methods as reported herein the multitude of mammalian cells is 1000 to 10,000,000 cells.

This analysis of a multitude of cells obtained from the same transfection allows the determination of the efficiency and specificity of the gene editing step.

In one embodiment of the methods as reported herein the method is for determining gene editing efficiency, for determining gene editing specificity, or for determining gene editing efficiency and specificity.

This analysis further allows, due to the possibility to perform a statistical analysis of the transfection results, to differentiate between homozygous and heterozygous gene modification as well as to differentiate between site specific and non-(site-)specific gene disruption and integration events.

In one embodiment of the methods as reported herein the method is for determining homozygous and heterozygous gene modification, or for determining site specific and non-(site-)specific gene disruption and integration.

A homozygous DPH gene inactivation causes toxin resistance. In this case all alleles of the DPH gene are inactivated in the cell.

In one embodiment of the methods as reported herein the introduction of the nucleic acid is at least a single introduction of the nucleic acid into the genome of the mammalian cell if the transfected cell is viable in the presence of the selection marker.

Toxin sensitivity differentiates homozygous and heterozygous target gene modifications as well as non-modification. Thus, in case the transfected cell is sensitive to the toxin but not to the selection marker then the transfection resulted in a heterozygous or non-specific gene integration.

In one embodiment of the methods as reported herein the introduction of the nucleic acid is a nucleic acid introduction into the genome of the mammalian cell if the transfected cell is not viable in the presence of the toxin but viable in the presence of the selection marker.

In one embodiment of the methods as reported herein the introduction of the nucleic acid is a nucleic acid knock-out (complete functional inactivation) in the genome of the mammalian cell if the transfected cell is viable in the presence of the toxin.

The resistance to the selection marker is a manifestation of the integration of the nucleic acid. If it is in the absence of toxin resistance then the integration took place at the position different from that of the DPH gene in the genome. If simultaneously the transfected cell is resistant to the toxin then the integration of the nucleic acid, without being bound by this theory, took place in the DPH gene resulting in its inactivation.

In one embodiment of the methods as reported herein DPH gene inactivation and nucleic acid integration events are quantified by a combination of cultivation in the presence of toxin and selection marker, and optionally high-resolution melting (HRM) PCR.

By comparing frequencies of toxin resistance, selection marker resistance, or double resistances a differentiation between integration and integration with inactivation can be made. In the first case it is a non-(site-)specific integration (nucleic acid integrated but target gene not inactivated), whereas in the second case it is, without being bound by this theory, a site-specific integration (nucleic acid integrated and target gene has been inactivated). Homozygous gene inactivation results in toxin resistance; targeted nucleic acid integration results in toxin as well as selection marker resistance; non-targeted integration events result only in selection marker resistance.

By comparing the frequencies of toxin, selection marker, and double resistances also a determination of the specificity and efficiency of the gene editing process can be made. Thus, with the method as reported herein it is possible to evaluate and rank different gene editing methods or likewise to evaluate and rank different elements used in the same/in one gene editing method.

Thus, the robust readout of the method as reported herein, i.e. the number of toxin or selection marker resistant colonies, can be applied to evaluate the influence of method variables, such as, e.g., sequence lengths of guide RNAs in CRISPR/Cas gene editing methods, on gene modification efficiency and type of modification.

In one embodiment of the methods as reported herein the method is for the evaluation/for comparing the efficiency of different gene editing methods and comprises the following steps
- transfecting the mammalian cell with one or more plasmids comprising the nucleic acid to be introduced, a nucleic acid conferring resistance to a selection marker, and the elements required for a first gene editing method for editing said DPH gene,
- cultivating the transfected cell in the absence of selection pressure,
- splitting the culture into at least two aliquots, or taking at least two samples from the culture,
- cultivating the first aliquot or sample in the presence of a DPH gene expression dependent toxin, and cultivating the second aliquot or sample in the presence of the corresponding selection marker,
- repeating these steps for all gene editing methods to be tested, and
- ranking the different gene editing methods based on the frequencies of toxin, selection marker, or double-resistances.

Diphtheria toxin ADP-ribosylates diphthamide on eEF2, thereby inactivating eEF2. This irreversibly stalls protein synthesis and kills cells. Diphthamide is a defined histidine modification, which generated by diphthamide synthesis genes, such as e.g. the diphthamide biosynthesis genes 1, 2, 4 and 5 (DPH1, DPH2, DPH4, and DPH5). The inactivation of these genes stops synthesis of toxin target and renders cells resistant to pseudomonas exotoxin A and diphtheria toxin (Stahl et al. 2015).

The frequency of the inactivation of all alleles of a target gene can be detected in a rapid and robust manner by counting toxin resistant colonies.

In one embodiment of the methods as reported herein the frequency of the (in vitro) inactivation of all alleles of a target gene in a cell is detected by counting toxin resistant colonies.

Cells, in which only one allele of a target gene has been modified, can be identified by HRM-PCR assays performed directly on cultured cells. Modification of the CRISPR/Cas target site alters the melting temperature of the respective DPH-gene derived PCR fragment compared to that of the wild-type-gene derived fragment. This is reflected by a bi-phasic melting curve in HRM profiles.

In one embodiment of the methods as reported herein the (in vitro) inactivation of one allele of a target gene in a cell is detected by HRM-PCR by the presence of a bi-phasic melting curve.

In one embodiment of the methods as reported herein the HRM-PCR is performed directly on cultured cells.

As CRISPR/Cas9 mediated gene-inactivation events are rarely identical on both (all) alleles in a cell, many cells with complete gene inactivation will also show bi-phasic HRM profiles. These can be differentiated from mono-allelic gene alterations by their toxin resistant phenotype.

In one embodiment of the methods as reported herein the frequency of the inactivation of all alleles of a target gene by CRISPR/Cas9 is detected by counting toxin resistant colonies in combination with a bi-phasic melting curve determined by HRM-PCR.

Thus, a combination of high throughput on-cell HRM-PCR and toxin selection (colony count) assays enables a quantification of heterozygous and homozygous DPH gene specific modification events.

The exemplary used puromycin-N-acetyl-transferase (PAC), which is encoded by an integration cassette of the applied CRISPR/Cas9 plasmids, inactivates puromycin (PM, selection marker) and hence renders cells PM resistant. As this is a general principle it will work with any antibiotic commonly used for the selection of positively transfected cells.

Thus, toxin resistance, e.g. pseudomonas exotoxin (PE) and diphtheria toxin (DT) resistance, results from specific and homozygous target gene inactivation.

Selection marker, e.g. PM, resistance is characteristic for any integration event, independent from the position of integration.

The frequencies of site specific vs. non-(site-)specific integration can be addressed by comparing number of selection marker (e.g. PM) resistant cells exposed to target gene specific guide RNA's and of cells that were exposed to scrambled non-specific guide RNAs.

In one embodiment of the methods as reported herein plasmids encoding DPH gene specific CRISPR/Cas9 modules were transfected into mammalian cells and the transfected cells were subsequently subjected to HRM-PCR as well as to colony count assays (to detect toxin (DT) and selection marker (PM) resistant cells), and wherein the method is for the determination of the frequency of site specific versus non-site-specific integration.

DPH gene inactivation showed absolute dependency on matching guide RNA sequence, wherein scrambled guide RNAs did not generate any DT-resistant colony. This indicates that CRISPRR/Cas9/DPH-guide-mediated PAC-gene integration occurs with preference at the DPH gene, but not with absolute specificity.

In one embodiment of the methods as reported herein the DPH gene is selected from the group consisting of the DPH1 gene, the DPH2 gene, the DPH4 gene, and the DPH5 gene.

Inactivation of both alleles of any of the DPH1 gene, the DPH2 gene, the DPH4 gene and the DPH5 gene confers absolute toxin resistance

In one embodiment of the methods as reported herein the method comprises the step of determining the number of toxin resistant colonies, the number of antibiotic resistant colonies, and the number of toxin and antibiotic resistant colonies, wherein the ratio between integration events (number of antibiotic resistant colonies) and inactivation events (number of toxin resistant colonies) is/reflects the specificity of the method.

In one embodiment of the methods as reported herein the method is for the selection of guide RNAs for CRISPR/Cas9 targeted integration of a nucleic acid, whereby the method comprises the steps of providing a multitude of different guide RNAs, and selecting the guide RNA that has the highest ratio between integration events (number of antibiotic resistant colonies) and inactivation events (number of toxin resistant colonies).

Frequencies of toxin-resistant colonies reflect target gene specific homozygous gene inactivation. Simultaneously, numbers of antibiotic resistant and toxin-antibiotic double resistant colonies were assessed to monitor cassette integration. The ratio between integration events (antibiotic resistance) and inactivation events (toxin resistance) can be used as a 'specificity indicator' to identify conditions at which specific integration occurs with at the same time the least gene inactivation events. Such conditions may be favored if one desired targeted integration without inflicting high numbers of non-productive target gene damage.

Low values (e.g. few antibiotic resistant colonies in relation to toxin resistant colonies) reflect inefficient integration in relation to simultaneous occurring inactivation events. High values (more antibiotic-resistant colonies and/or relatively decreased numbers of toxin-resistant colonies) reflect non-specific cleavage at CRISPR/Cas9 affected target genes.

Shorter guide RNAs improve not only the integration efficiency (higher overall numbers of antibiotic-resistant colonies), but also the ratio between productive and non-productive gene editing (reduction in toxin-resistant colonies without insertion).

In one embodiment of the methods as reported herein the gene editing method is selected from the group consisting of CRISPR/Cas, zinc finger nuclease, and TALEN.

The gene editing modules and parameters can be optimized by DPH gene modification and thereafter transferred to optimize gene editing efficiency or specificity of other genes.

Thus, 20mers may be the choice if one aims for most efficient gene inactivation, 16-18mers may be preferred if one desires integration without excessive destructive editing

If one aims for re-application of CRISPR/Cas modules to previously treated cells to increase integration efficiency the herein reported method of determining the most suitable guide RNAs as gene inactivation alters the guide RNA target sequence can be used. Thus, only unaltered genes can be modified by the original CRISPR/Cas9 components while modified genes (without integration) are not susceptible to the first applied approach using the same guide RNAs.

One aspect as reported herein is a method for the identification/selection of (mutated versions or variants of) CRISPR/Cas9 or ZFNs or TALENs or other gene editing modules comprising the following steps
- providing/preparing a multitude of variants of one or more gene editing modules,
- determining the efficiency and/or highest ratio between integration events (number of antibiotic-resistant colonies) and inactivation events (number of toxin-resistant colonies) with a method as reported herein, and
- identifying/selecting the variant that has the highest efficiency and/or highest ratio.

One aspect as reported herein is a method for the selection of compounds or compound combinations that modify (enhance or reduce) the efficiency or specificity of a gene editing module/method comprising the following steps
- providing one or more compound or one or more combination of compounds,
- optionally determining the efficiency and/or ratio between integration events (number of antibiotic-resistant colonies) and inactivation events (number of toxin-resistant colonies) with a method as reported herein in the absence of said compounds or combination of compounds,
- determining for each of said compounds or combination of compounds separately/individually the efficiency and/or ratio between integration events (number of antibiotic-resistant colonies) and inactivation events (number of toxin-resistant colonies) with a method as reported herein in the presence of said compounds or combination of compounds,
- identifying/selecting at least one compounds or combination of compounds that has an efficiency and/or ratio that is different from the efficiency and/or ration of the method as reported herein performed in the absence of said compounds or combination of compounds.

One aspect as reported herein is a method for the determination of compound concentrations and time points of addition thereof to enhance the efficiency or specificity of a gene editing method while minimizing growth inhibition or toxicity comprising the following steps
- providing one or more compound or one or more combination of compounds,
- optionally determining the efficiency and/or ratio between integration events (number of antibiotic-resistant colonies) and inactivation events (number of toxin-resistant colonies) with a method as reported herein in the absence of said compounds or combination of compounds,
- determining for each of said compounds or combination of compounds separately/individually the efficiency and/or ratio between integration events (number of antibiotic-resistant colonies) and inactivation events (number of toxin-resistant colonies) with a method as reported herein in the presence of said compounds or combination of compounds at different concentration and/or time points of addition,
- identifying/selecting for each of the at least one compounds or combination of compounds a concentration and/or time point of addition that has a higher efficiency and/or higher ratio than that of the method as reported herein performed in the absence of said compounds or combination of compounds.

In one embodiment of the methods reported before is the identifying/selecting of the compound that has the highest efficiency and/or highest ratio.

### Detailed Description of the Invention

### Definitions

It must be noted that as used herein and in the appended claims, the singular forms "a", "an", and "the" include plural reference unless the context clearly dictates otherwise. Thus, for example, reference to "a cell" includes a plurality of such cells and equivalents thereof known to those skilled in the art, and so forth. As well, the terms "a" (or "an"), "one or more" and "at least one" can be used interchangeably herein.

It is also to be noted that the terms "comprising", "including", and "having" can be used interchangeably and include the term "consisting".

To a person skilled in the art procedures and methods are well known to convert an amino acid sequence, e.g. of a polypeptide, into a corresponding nucleic acid sequence encoding this amino acid sequence. Therefore, a nucleic acid is characterized by its nucleic acid sequence consisting of individual nucleotides and likewise by the amino acid sequence of a polypeptide encoded thereby.

The term "about" denotes a range of +/- 20 % of the thereafter following numerical value. In one embodiment the term about denotes a range of +/- 10 % of the thereafter following numerical value. In one embodiment the term about denotes a range of +/- 5 % of the thereafter following numerical value.

Pseudomonas Exotoxin A (PE), Diphtheria Toxin (DT), Cholix Toxin (CT), and related toxins are bacterial proteins that ADP-ribosylate the Diphthamide residue of eukaryotic translation elongation factor A (eEF2). Using NAD as a co-substrate, ADP is transferred to the Diphthamide of eEF2. This inactivates the functionality of eEF2. Cells with ADP-ribosylated eEF2 stall their protein synthesis and thereby die. PE, DT, CT, and related toxins require the presence of Diphthamide on eEF2 to ADP-ribosylate eEF2. EEf2 without diphthamide cannot be ADP-ribosylated by these toxins.

The terms "cell", "cell line", and "cell clone" are used interchangeably and refer to cells into which exogenous nucleic acid has been introduced, including the progeny of such cells. "Cells" include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

A "plasmid" is a nucleic acid providing all required elements for the expression of the comprised structural gene(s) in a host cell. Typically, an expression plasmid comprises a prokaryotic plasmid propagation unit, e.g. for E. coli, comprising an origin of replication, and a selectable marker, an eukaryotic selection marker, and one or more expression cassettes for the expression of the structural gene(s) of interest each comprising a promoter, a structural gene, and a transcription terminator including a polyadenylation signal. Gene expression is usually placed under the control of a promoter, and such a structural gene is said to be "operably linked to" the promoter. Similarly, a regulatory element and a core promoter are operably linked if the regulatory element modulates the activity of the core promoter. The term "plasmid" includes e.g. shuttle and expression plasmids as well as transfection plasmids.

A "selection marker" is a nucleic acid that allows cells carrying the selection marker to be specifically selected for or against, in the presence of a corresponding selection agent. Typically, a selection marker will confer resistance to a drug or compensate for a metabolic or catabolic defect in the host cell. A selection marker can be positive, negative, or bifunctional. A useful positive selection marker is an antibiotic resistance gene. This selection marker allows the host cell transformed therewith to be positively selected for in the presence of the corresponding selection agent, e.g. the antibiotic. A non-transformed host cell is not capable to grow or survive under the selective conditions, i.e. in the presence of the selection agent, in the culture. Positive selection markers allow selection for cells carrying the marker, whereas negative selection markers allow cells carrying the marker to be selectively eliminated. Selection markers used with eukaryotic cells include, e.g., the genes for aminoglycoside phosphotransferase (APH), such as e.g. the hygromycin (hyg), neomycin (neo), and G418 selection markers, dihydrofolate reductase (DHFR), thymidine kinase (tk), glutamine synthetase (GS), asparagine synthetase, tryptophan synthetase (selection agent indole), histidinol dehydrogenase (selection agent histidinol D), and nucleic acids conferring resistance to puromycin, bleomycin, phleomycin, chloramphenicol, Zeocin, and mycophenolic acid. Further marker genes are described e.g. in WO 92/08796 and WO 94/28143.

### Detailed Description

Prerequisite for optimizing gene editing is the reliable and robust detection and differentiation of heterozygous and homozygous gene inactivation as well as non-specific and targeted integration events. Existing methods such as determination of phenotypes caused by insertions (e.g. drug resistances) or lack of phenotypes (gene inactivation) or sequencing approaches do frequently not differentiate homozygous and heterozygous inactivations. Moreover, existing technologies rarely address genetic composition of individual cells or are not based on large numbers of individual gene edited cells to allow robust statistical analyses.

Off-target effects created by site-specific nucleases can be toxic to cells, and difficult to predict and monitor comprehensively. Complex genomes, however, often contain multiple copies of sequences that are identical or highly homologous to the intended DNA target, leading to off-target activity and cellular toxicity (Gaj, T., et al., Trends Biotechnol. 31 (2013) 397-405).

Herein is reported a simple and robust approach to characterize gene editing events. A combination of DPH gene inactivation, toxin treatment and (antibiotic) selection marker selection allows the determination of gene editing efficacies on very large numbers of individual cells. With the method it is possible to differentiate between homo- and heterozygous gene inactivation as well as site-specific and non-site-specific integration. The simplicity and robustness of the method as reported herein can be used amongst other things in the optimization of gene editing procedures and modules, as well as in the identification and comparison of gene editing modulators.

Efficacies and specificities of, e.g., CRISPR/Cas9-mediated or zinc finger nuclease (ZFN)-mediated homozygous and heterozygous gene inactivation and cassette integration events have been quantified with the method as reported herein by a combination of toxin (e.g. diphtheria toxin (DT)) and (antibiotic) selection marker (e.g. puromycin (PM)) selection and high-resolution melting (HRM) PCR.

Overall gene inactivation frequencies can be detected by HRM-PCR: homozygous DPH1 or DPH2 gene inactivation causes toxin resistance (e.g. diphtheria toxin resistance (DTr)), homozygous and heterozygous DPH modifications can hence be differentiated by toxin sensitivity. Selection marker resistance (e.g. puromycin resistance (PMr)) is caused by expression cassette integration.

Target gene specific colony counts can differentiate homo- or heterozygous inactivation and integration events in 10⁴-10⁵ individual cells per experiment, assessing hundreds of individual clones harboring inactivated genes in one experiment.

It has been found that homozygous inactivation (DTr) occurs with ~30-50 fold higher frequency than targeted cassette integration (DTr & PMr) or non-targeted integration (PMr with scrambled RNA (scRNA)).

It has been found that heterozygous gene inactivation without integration occurred more than 100 fold more frequent than integration.

Preference for gene inactivation over integration is independent of target sequence, gene or chromosomal location.

It has been found that colony counts can address variables incl. guide RNA (gRNA) length, choice of enzymes for gene editing, or modulators of non-homologous end-joining (NHEJ) or homologous recombination (HR).

With the method as reported herein it has been found that 20mer gRNAs were most effective for inactivation, whereas 16-18mer gRNAs provided highest integration efficacies.

With the method as reported herein it has been found that non-modified CRISPR/Cas9 was twice as efficient as ZFN-editing and 5 fold more efficient than engineered high fidelity CRISPR/Cas9, regarding gene inactivation as well as integration. It has been found that ratios between inactivation, non-targeted and targeted integration events were similar for the different enzymes.

The method s reported herein is also suitable to address effects of NHEJ or HR modulation on editing efficiency and specificity.

Thus, a method for assessing the above is beneficial.

### The diphthamide modification

Stahl, S., et al. (Proc. Natl. Acad. Sci. USA 112 (2015) 10732-10737) reported that eukaryotic translation elongation factor 2 (eEF2) is a highly conserved protein and essential for protein biosynthesis. The diphthamide modification at His715 of human eEF2 (or at the corresponding position in other species) is conserved in all eukaryotes and in archaeal counterparts. It is generated by proteins that are encoded by seven genes. Proteins encoded by diphthamide biosynthesis protein 1 (DPH1), DPH2, DPH3, and DPH4 (DNAJC24) attach a 3-amino-3-carboxypropyl (ACP) group to eEF2. This intermediate is converted by the methyl-transferase DPH5 to diphthine, which is subsequently amidated to diphthamide by DPH6 and DPH7.

Diphthamide-modified eEF2 is the target of ADP ribosylating toxins, including pseudomonas exotoxin A (PE) and diphtheria toxin (DT). These bacterial proteins enter cells and catalyze ADP ribosylation of diphthamide using nicotinamide adenine dinucleotide (NAD) as substrate. This inactivates eEF2, arrests protein synthesis, and kills the cell.

In Stahl et al. gene-specific zinc finger nucleases (ZFN) were applied to generate MCF7 cells with inactivated DPH genes.

Therefore plasmids encoding ZFNs were transfected into MCF7 cells. Forty-eight hours after transfection, in order to enable ZFN binding, double-strand breaks and mis-repair, mutated cells were identified by either phenotype selection or by genetic analyses. For phenotype selection, cells were exposed to lethal doses of PE (100 nM) to kill all cells whose eEF2 is a substrate for the toxin. After an additional 48 hours, dead cells were removed and the culture propagated in toxin-containing media. This procedure generated colonies of cells transfected with ZFNs for DPH1, DPH2, DPH4 and DPH5. No colonies were obtained under toxin selection in cells that were mock transfected, or with ZFNs that target DPH3, DPH6 and DPH7.

To identify MCF7 mutants without toxin selection, single cells from each transfection were subjected to high-resolution melting (HRM) analyses genes. This technique identifies cells that contain two different alleles of the gene to be analyzed, as those generate biphasic or odd-shaped melting curves. Analyses of candidate clones with bi-phasic HRM profiles confirmed the presence of different DPH allele sequences. This approach delivered clones that had one gene copy inactivated and another functional wild-type copy for all DPH genes.

In wild-type cells, only diphthamide-modified eEF2 is detectable without evidence for unmodified eEF2 or diphthine or ACP modifications. Cells with complete inactivation of the DPH1, DPH2, DPH4 as well as DPH5 genes contained no diphthamide-modified eEF2. Thus, these genes are essential for diphthamide synthesis and other genes cannot compensate their inactivation. Complete inactivation of DPH1, DPH2 or DPH4 generated cells in which only unmodified eEF2 and no other modified form was detectable. Complete inactivation of DPH5 generated the ACP intermediate (eEF2 with this intermediate is not recognized by the antibody applied in the preceding Western blots). The major eEF2 species in cells with one inactivated and one functional copy of DPH1 to DPH7 is diphthamide-modified eEF2.

EEF2 of parental MCF7, and of all seven heterozygote-inactivated MCF7 derivatives (DPH1-7) becomes ADP ribosylated by PE. In contrast, eEF2 from cells that have completely inactivated DPH1 or DPH2 or DPH4 or DPH5 genes is not amenable to ADP ribosylation. Only eEF2 with diphthamide, but not without modification (DPH1, DPH2, DPH4) or with partial modification (ACP in DPH5) serves as substrate for ADP ribosylating toxins.

Under normal growth conditions, no impact of DPH inactivation on growth for all heterozygous clones was observed. In addition, complete inactivation of DPH1, DPH2 or DPH4 did not cause significant reductions in cell growth or viability. Cells with complete inactivation of DPH1, DPH2 and DPH4 harbor only unmodified eEF2. Thus, the exclusive presence of unmodified eEF2 by itself does not inhibit the growth of MCF7. Reduced growth rates were observed for all clones with completely inactivated DPH5.

Cells with complete inactivation of DPH1, DPH2, DPH4 or DPH5 show increased TNF sensitivity. NF-κB and death receptor signaling pathways (known to be involved in and necessary for development) are pre-activated in diphthamide-deficient cells. These cells are nevertheless viable, as pathway induction does not pass thresholds sufficient to induce apoptosis without additional stimuli. Pre-sensitization becomes phenotypically relevant upon triggering these pre-induced pathways: all diphthamide synthesis-deficient cells (independent from target gene knockout) were hypersensitive to TNF-induced apoptosis. This finding indicates that the presence or absence of diphthamide affects NF-κB or death receptor pathways.

The DPH genes have the nucleotide sequence as deposited in NM_001383.3 (DPH1), NC_000001.11 (DPH2), NC_000003.12 (DPH3), NM_181706.4 (DPH4), BC053857.1 (DPH5), NM _080650.3 (DPH6) and NC_000009.12 (DPH7). Mutated clones were either obtained by isolating survivor clones following exposure to lethal doses of Pseudomonas exotoxin A, or by PCR-based HRM analyses.

Thus, in one embodiment of the method as reported herein for toxin selection, cells were treated 48 h after transfection with 100 nM PE and further propagated to generate toxin-resistant colonies, these colonies were isolated and re-cloned from single cells, and for genetic screen, gene-specific PCR fragments were generated and subjected to HRM (marking mutation containing clones by biphasic melting curves).

In one embodiment growth of parental and mutated MCF-7 is assessed by seeding 10,000 cells in flat-bottom 96-well plates and incubation at 37 °C in humidified 5% CO₂, exposing cells twenty-four hours after seeding to toxin, determining cell growth, and performing a cell proliferation assay (e.g. CellTiterGlo 96 Aqueous One Solution Cell Proliferation Assay, Promega, according to the manufacturer's instructions, wherein proliferation (DNA replication) is addressed by BrdU incorporation assays (Roche Diagnostics, Mannheim FRG) 72 h after toxin exposure).

**Definitions** (adopted from Gaj, T., et al., Trends Biotechnol. 31 (2013) 397-405):
CRISPR/Cas (CRISPR associated) systems: clustered regulatory interspaced short palindromic repeats are loci that contain multiple short direct repeats, and provide acquired immunity to bacteria and archaea. CRISPR systems rely on crRNA and tracrRNA for sequence-specific silencing of invading foreign DNA. Three types of CRISPR/Cas systems exist: in type II systems, Cas9 serves as an RNA-guided DNA endonuclease that cleaves DNA upon crRNA-tracrRNA target recognition.
crRNA: CRISPR RNA base pairs with tracrRNA to form a two-RNA structure that guides the Cas9 endonuclease to complementary DNA sites for cleavage.
PAM: protospacer adjacent motifs are short nucleotide motifs that occur on crRNA and are specifically recognized and required by Cas9 for DNA cleavage.
tracrRNA: trans-activating chimeric RNA is noncoding RNA that promotes crRNA processing and is required for activating RNA-guided cleavage by Cas9.
DSB: the product of ZFN, TALEN, and CRISPR/Cas9 action, double-strand breaks are a form of DNA damage that occurs when both DNA strands are cleaved.
HR: homology-directed repair is a template-dependent pathway for DSB repair. By supplying a homology-containing donor template along with a site- specific nuclease, HDR faithfully inserts the donor molecule at the targeted locus. This approach enables the insertion of single or multiple transgenes, as well as single nucleotide substitutions.
NHEJ: non-homologous end joining is a DSB repair pathway that ligates or joins two broken ends together. NHEJ does not use a homologous template for repair and thus typically leads to the introduction of small insertions and deletions at the site of the break, often inducing frame-shifts that knockout gene function.
TALENs: transcription activator-like effector nucleases are fusions of the FokI cleavage domain and DNA-binding domains derived from TALE proteins. TALEs contain multiple 33-35-amino-acid repeat domains that each recognizes a single base pair. Like ZFNs, TALENs induce targeted DSBs that activate DNA damage response pathways and enable custom alterations.
ZFNs: zinc-finger nucleases are fusions of the nonspecific DNA cleavage domain from the FokI restriction endonuclease with zinc-finger proteins. ZFN dimers induce targeted DNA DSBs that stimulate DNA damage response pathways. The binding specificity of the designed zinc-finger domain directs the ZFN to a specific genomic site.
ZFNickases: zinc-finger nickases are ZFNs that contain inactivating mutations in one of the two FokI cleavage domains. ZFNickases make only single-strand DNA breaks and induce HDR without activating the mutagenic NHEJ pathway.

### Gene editing methods

Approach enabling the manipulation of virtually any gene in a diverse range of cell types and organisms have evolved during the past decades. This core technology - commonly referred to as 'genome editing' - is based on the use of engineered nucleases composed of sequence-specific DNA-binding domains fused to a nonspecific DNA cleavage module. These chimeric nucleases enable efficient and precise genetic modifications by inducing targeted DNA double-strand breaks (DSBs) that stimulate the cellular DNA repair mechanisms, including error-prone non-homologous end joining (NHEJ) and homology-directed repair (HDR). The versatility of this approach is facilitated by the programmability of the DNA-binding domains.

The versatility of these methods arises from the ability to customize the DNA-binding domain to recognize virtually any sequence.

Thus, the ability to execute genetic alterations depends largely on the DNA-binding specificity and affinity of the designed proteins (Gaj, T., et al., Trends Biotechnol. 31 (2013) 397-405).

Two types of gene-specific manipulations can be envisioned: non-specific, non-targeted mutagenesis and targeted gene modification or gene replacement.

Unspecified mutagenic agents are targeted to one gene. The outcome of targeted mutagenesis is a localized sequence alteration.

Targeted gene replacement produces by homologous recombination be- tween the original and exogenous gene copies localized sequence changes. In targeted gene replacement, the goal is to replace an existing sequence with one designed in the laboratory. The latter allows the introduction of both more subtle and more extensive alterations. Making directed genetic changes is often called "gene targeting." (Carroll, D., Genetics, 188 (20111) 773-782).

Zinc-finger nucleases (ZFNs) and transcription activator-like effector nucleases (TALENs) as well as CRISPR/Cas represent a powerful class of tools that are redefining the boundaries of biological research. These chimeric nucleases are composed of programmable, sequence-specific DNA-binding modules linked to a nonspecific DNA cleavage domain. ZFNs and TALENs enable a broad range of genetic modifications by inducing DNA double-strand breaks that stimulate error-prone non-homologous end joining or homology-directed repair at specific genomic locations. Clustered regulatory interspaced short palindromic repeat (CRISPR)/Cas-based RNA-guided DNA endonucleases rely on crRNA and tracrRNA for sequence-specific modification of DNA. Three types of CRISPR/Cas systems exist: in type II systems, Cas9 serves as an RNA-guided DNA endonuclease that cleaves DNA upon crRNA-tracrRNA target recognition.

By co-delivering a site-specific nuclease with a donor plasmid bearing locus-specific homology arms, single or multiple transgenes can be efficiently integrated into an endogenous locus. In addition to their role in facilitating HR, site-specific nucleases also allow rapid generation of cell lines and organisms with null phenotypes; NHEJ-mediated repair of a nuclease-induced DSB leads to the introduction of small insertions or deletions at the targeted site, resulting in knockout of gene function via frame shift mutations. Site- specific nucleases can also induce deletions of large chromosomal segments. This method has been shown to support large chromosomal inversions and translocations. Finally, by synchronizing nuclease-mediated cleavage of donor DNA with the chromosomal target, large transgenes (up to 14 kb) have been introduced into various endogenous loci via NHEJ-mediated ligation (Gaj, T., et al., Trends Biotechnol. 31 (2013) 397-405).

NHEJ-mediated repair of a nuclease-induced DSB leads to the efficient introduction of variable length insertion/deletion (indel) mutations that originate at the site of the break. Thus, NHEJ-mediated repair of DSBs introduced into gene coding sequences will often yield frame shift mutations that can lead to knockout of gene function.

If a double-stranded DNA "donor template" is supplied, HR of a nuclease- induced DSB can be used to introduce precise nucleotide substitutions or insertions of up to 7.6 kb at or near the site of the break. Recent work has also shown that oligonucleotides can be used with ZFNs to introduce precise alterations, small insertions, and large deletions. ZFNs have been used to introduce NHEJ- or HR-mediated gene alterations (Joung, J.K. and Sander, J.D., Nat. Rev. Mol. Cell Biol. 14 (2013) 49-55).

Typically, nuclease-encoded genes are delivered into cells by plasmid DNA, viral vectors, or in vitro transcribed mRNA. Transfection of plasmid DNA or mRNA by electroporation or cationic lipid-based reagents can be toxic and restricted to certain cell types. Viral vectors also present limitations, because they are complex, difficult-to-produce, potentially immunogenic, and involve additional regulatory hurdles. Integrase-deficient lentiviral vectors (IDLVs) are an attractive alternative for delivering ZFNs into transfection-resistant cell types. AAV is a promising vector for ZFN delivery that has been used to enhance the efficiency of ZFN-mediated HR and drive ZFN-mediated gene correction in vivo. Although adenoviral vectors can accommodate and deliver full-length TALEN genes into human cells, lentiviral plasmid vectors harboring TALEN sequences are prone to rearrangements after transduction (Gaj, T., et al., Trends Biotechnol. 31 (2013) 397-405).

### Zink-finger-nucleases (ZFN)

Zink-finger-nucleases that combine the non-specific cleavage domain (N) of FokI endo-nuclease with zinc finger proteins (ZFPs) offer a general way to deliver a site-specific double-strand break (DSB) to the genome.

The modular structure of zinc finger (ZF) motifs and modular recognition by ZF domains make them the versatile DNA recognition motifs for designing artificial DNA-binding proteins. Each ZF motif consists of approx. 30 amino acids and folds into ßßa structure, which is stabilized by chelation of a zinc ion by the conserved Cys2His2 residues. The ZF motifs bind DNA by inserting the a-helix into the major groove of the DNA double helix. Each finger primarily binds to a triplet within the DNA substrate. Key amino acid residues at positions -1, +1, +2, +3, +4, +5 and +6 relative to the start of the a-helix of each ZF motifs contribute to most of the sequence-specific interactions with the DNA site. These amino acids can be changed while maintaining the remaining amino acids as a consensus backbone to generate ZF motifs with different triplet sequence-specificities. Binding to longer DNA sequences is achieved by linking several of these ZF motifs in tandem to form ZFPs. The designed ZFPs provide a powerful platform technology since other functionalities like non-specific FokI cleavage domain (N), transcription activator domains (A), transcription repressor domains (R) and methylases (M) can be fused to a ZFPs to form ZFNs respectively, zinc finger transcription activators (ZFA), zinc finger transcription repressors (ZFR) and zinc finger methylases (ZFM).

FokI restriction enzyme, a bacterial type IIS restriction endo-nuclease recognizes the non-palindromic penta deoxy-ribonucleotide, 5'-GGATG-3' : 5'-CATCC-3', in duplex DNA and cleaves 9/13 nt downstream of the recognition site. Durai et al. suggested that it is possible to swap the FokI recognition domain with other naturally occurring DNA-binding proteins that recognize longer DNA sequences or other designed DNA-binding motifs to create chimeric nucleases (Durai, S., et al., Nucl. Acids Res. 33 (2005) 5978-5990).

The FokI nuclease functions as a dimer and therefore two zinc finger arrays must be designed for each target site. Early ZFNs used wild-type homodimeric FokI domains, which can form unwanted dimers of the same monomeric ZFN. The use of obligate heterodimeric FokI domains reduce the formation of unwanted homodimeric species and therefore have improved specificities (Joung, J.K. and Sander, J.D., Nat. Rev. Mol. Cell Biol. 14 (2013) 49-55). Thus, a ZFN target sites consist of two zinc-finger binding sites separated by a 5-7-bp spacer sequence recognized by the FokI cleavage domain (Gaj, T., et al., Trends Biotechnol. 31 (2013) 397-405).

Plasmids for one-hybrid genetic selection system: The reporter gene, either chloramphenicol acetyltransferase (CAT), or GFP is located downstream from a weak lac derivative promoter (Pwk) on pDB series plasmids. A 9 bp target site for binding by the ZF is located at a specific distance from the start of transcription.

On the pA series of plasmids, the gene for the ZF is fused to a fragment of the a-subunit of RNA polymerase (rpoA[1-248]) via a sequence coding for an amino acid linker. Binding of the rpoA[1-248]-ZF fusion to the 9 bp site in the reporter plasmid recruits the other RNA polymerase subunits to stimulate transcription of the reporter gene (Durai, S., et al., Nucl. Acids Res. 33 (2005) 5978-5990).

### Transcription activator-like effector nucleases (TALENs)

Fusions of transcription activator-like (TAL) effectors of plant pathogenic Xanthomonas spp. to the FokI nuclease, TALENs bind and cleave DNA in pairs. Binding specificity is determined by customizable arrays of polymorphic amino acid repeats in the TAL effectors.

TAL effectors enter the nucleus, bind to effector-specific sequences in host gene promoters and activate transcription. Their targeting specificity is determined by a central domain of tandem, 33-35 amino acid repeats, followed by a single truncated repeat of 20 amino acids. Naturally occurring recognition sites are uniformly preceded by a T that is required for TAL effector activity (Cermak, T., et al., Nucl. Acids Res. 39 (2011) e82).

TALE specificity is determined by two hypervariable amino acids that are known as the repeat-variable di-residues (RVDs). Like zinc fingers, modular TALE repeats are linked together to recognize contiguous DNA sequences (Gaj, T., et al., Trends Biotechnol. 31 (2013) 397-405).

TAL effectors can be fused to the catalytic domain of the FokI nuclease to create targeted DNA double-strand breaks (DSBs) in vivo for genome editing. Since FokI cleaves as a dimer, these TAL effector nucleases (TALENs) function in pairs, binding opposing targets across a spacer over which the FokI domains come together to create the break. DSBs are repaired in nearly all cells by one of two highly conserved processes, non-homologous end joining (NHEJ), which often results in small insertions or deletions and can be harnessed for gene disruption, and homologous recombination (HR), which can be used for gene insertion or replacement.

Assembly of a TALEN or TAL effector construct involves two steps: (i) assembly of repeat modules into intermediary arrays of 1-10 repeats and (ii) joining of the intermediary arrays into a backbone to make the final construct (Cermak, T., et al., Nucl. Acids Res. 39 (2011) e82).

TALEN target sites consist of two TALE binding sites separated by a spacer sequence of varying length (12-20 bp) (Gaj, T., et al., Trends Biotechnol. 31 (2013) 397-405).

For typical heterodimeric target sites (i.e. such as would typically occur in a native DNA sequence), paired TALEN constructs are transformed together into the target cell.

One of the pairs of TALENs targeting the human gene of interest is subcloned into a mammalian expression vector using suitable restriction endonucleases. These enzymes excise the entire TALEN pair and place the coding sequence under control of a promoter. The resulting plasmids were introduced into HEK293T cells by transfection using LipofectAmine 2000 (Invitrogen) following the manufacturer's protocol. Cells were collected 72 h after transfection (Cermak, T., et al., Nucl. Acids Res. 39 (2011) e82).

### Clustered Regularly Interspaced Short Palindromic Repeats/CRISPR-associated protein 9 (CRISPR/Cas9)

The naturally occurring CRISPR/Cas Type II system has been developed into powerful genetic editing tool for eukaryotic cells. Particularly the demonstration that crRNA and tracrRNA can be combined in to a single guide RNA (sgRNA) paved the way for this development. Cas9 produces a single double-stranded break in the DNA, an important feature of a gene-editing tool. The method makes use of DNA repair pathways in eukaryotic cells to provide two ways to make genetic alterations. The first relies on Non-Homologous End Joining (NHEJ) that joins the cut ends but in the process often deletes a few bases, which may cripple the gene product, or causes a frame shift that inactivates it. In the second, Homology Directed Repair (HDR) is used to repair the damaged allele using another piece of DNA with homology to the target. By providing a DNA element that can be inserted by recombination, any type of insertion, deletion or change in sequence can be achieved. The main limitation is the need for a PAM adjacent to the target. Off-target effects, where Cas9 interacts with an unintended target, are a concern requiring strategies for prediction and prevention (Rath, D., et al., Biochim. 117 (2015) 119-128).

In the type II CRISPR/Cas system, short segments of foreign DNA, termed 'spacers' are integrated within the CRISPR genomic loci and transcribed and processed into short CRISPR RNA (crRNA). These crRNAs anneal to trans-activating crRNAs (tracrRNAs) and direct sequence-specific cleavage and silencing of pathogenic DNA by Cas proteins. Recent work has shown that target recognition by the Cas9 protein requires a 'seed' sequence within the crRNA and a conserved dinucleotide-containing protospacer adjacent motif (PAM) sequence upstream of the crRNA- binding region. The CRISPR/Cas system has been shown to be directly portable to human cells by co-delivery of plasmids expressing the Cas9 endo-nuclease and the necessary crRNA components (Gaj, T., et al., Trends Biotechnol. 31 (2013) 397-405).

### High-resolution melting method of small amplicons for determining homo- and heterozygotes

Liew, M., et al. (Clinical Chemistry 50 (2004) 1156-1164) reported a high-resolution melting method of small amplicons for determining homo- and heterozygotes. Heterozygotes were easily identified because hetero-duplexes altered the shape of the melting curves. Adding 15% of a known homozygous genotype to unknown samples allows melting curve separation of all three genotypes.

Melting analysis of short PCR products in the presence of the hetero-duplex-detecting dye, LCGreen I, was used to genotype SNPs.

Although the heterozygotes can be identified by the presence of a second, low-temperature melting transition even with standard techniques, genotype differentiation is much easier with high-resolution methods.

PCR was performed in a LightCycler with reagents commonly used in clinical laboratories. The 10 µL reaction mixtures consisted of 10-50 ng of genomic DNA, 3 mM MgCl₂, 1 × LightCycler FastStart DNA Master Hybridization Probes master mixture, 1 × LCGreen I, 0.5 µM forward and reverse primers, and 0.01 U/µL Escherichia coli uracil N-glycosylase (UNG; Roche). The PCR was initiated with a 10 min. hold at 50 °C for contamination control by UNG and a 10 min. hold at 95 °C for activation of the polymerase. Rapid thermal cycling was performed between 85 °C and the annealing temperature at a programmed transition rate of 20 °C/s.

Melting analysis was performed on the LightCycler immediately after cycling. Twenty samples were analyzed at once by first heating to 94 °C, cooling to 40 °C, heating again to 65 °C (all at 20 °C/s), and then melting at 0.05 °C/s with continuous acquisition of fluorescence until 85 °C. LightCycler software was used to calculate the derivative melting curves.

When high-resolution melting was used, amplified samples were heated to 94 °C in the LightCycler and rapidly cooled to 40 °C. The LightCycler capillaries were then transferred to the HR-1 high-resolution instrument and heated at 0.3 °C/s. Samples were analyzed between 65 and 85 °C with a turnaround time of 1-2 min. High-resolution melting data were analyzed with HR-1 software. In most cases, plots of fluorescence versus temperature were normalized. For direct comparison with LightCycler data, derivative plots were used without normalization. All curves were plotted using Microsoft Excel after export of the data.

### The method as reported herein

Gene editing technologies are required for the generation of genetically modified tools, cells (cell lines) or organisms. To be suitable for routine application the technology has to have a high specificity and high efficiency but at the same time a low level of non-targeted gene modification, i.e. side reaction.

Herein is reported a high throughput method for determining and thereby comparing/ranking specificity of gene editing, efficiency of gene editing, and/or level of non-targeted gene editing of a gene editing method/gene editing modules.

Herein is reported a robust, high throughput method for quantification of a gene editing method/gene editing module, such as e.g. CRISPR/Cas, mediated gene alterations. The method is suitable for the differentiation between efficiency, site specific and non-(site-)specific gene disruption, and sequence integration events of a gene editing method/gene editing module (e.g. quantification of CRISR/Cas mediated gene inactivation and integration events).

In one embodiment of the methods as reported herein the gene editing method/module/technology is selected from the group consisting of CRISPR/Cas, zinc finger nuclease and talon nuclease.

In one embodiment of the methods as reported herein, the toxin is an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

### Exemplary embodiments

The method as reported herein is exemplified in the following using exemplary sequences and targets. This shall not be treated as a limitation of the current invention. It is provided merely as one of a multitude of alternatives, i.e. of specific embodiments, and as an evidence for the functioning of the herein reported method.

In one embodiment of the methods as reported herein the robust and simple method to quantify and optimization gene editing approaches as reported herein employs the combination of DPH gene inactivation, in a preferred embodiment DPH1 or DPH2 gene inactivation, combined with diphtheria toxin and/or puromycin selection. It is possible with this method not only to determine gene editing efficiency on large numbers of individual cells, i.e. in high throughput, but also to differentiate between homozygous and heterozygous gene as well as between site specific and non-(site-)specific gene disruption and integration events.

CRISPR/Cas mediated homozygous and heterozygous DPH gene inactivation and cassette integration events were quantified by a combination of diphtheria toxin (DT) and puromycin (PM) selection and high-resolution melting (HRM) PCR. DPH gene inactivation can be detected by high-resolution melting HRM-PCR and homozygous DPH gene inactivation causes toxin resistance. Thus, toxin sensitivity differentiates homozygous and heterozygous target gene modifications. PM resistance detects integration of the CRIPR/Cas cassettes. Comparing frequencies of DT-, PM- or double-resistant clones/colonies, it has been observed that homozygous gene inactivation (DT resistance) occurs at approx. 30-50 fold higher frequency than targeted cassette integration (DT as well as PM resistance) or non-targeted integration events (PM resistance). Heterozygous target gene inactivation without cassette integration (detectable by HRM-PCR) occurred at even higher (>100 fold) frequency than cassette integration. The preference for and higher incidence of gene inactivation over cassette integration is independent of the CRISPR/Cas9 target sequence, chromosomal location or target gene. The robust readout of the method as reported herein (number of DT or PM resistant colonies) can be applied to evaluate the influence of CRISPR/Cas9 variables such as sequence lengths of guide RNAs on gene modification efficiency and type of modifications. The results presented herein also imply that CRISPR/Cas9 derived therapies may be more suited for gene inactivation approaches than for applications that require targeted integration events.

### Exemplary Results

### Diphtheria toxin resistance assays and HRM-PCR quantify and differentiate between homozygous and heterozygous DPH1 gene inactivation

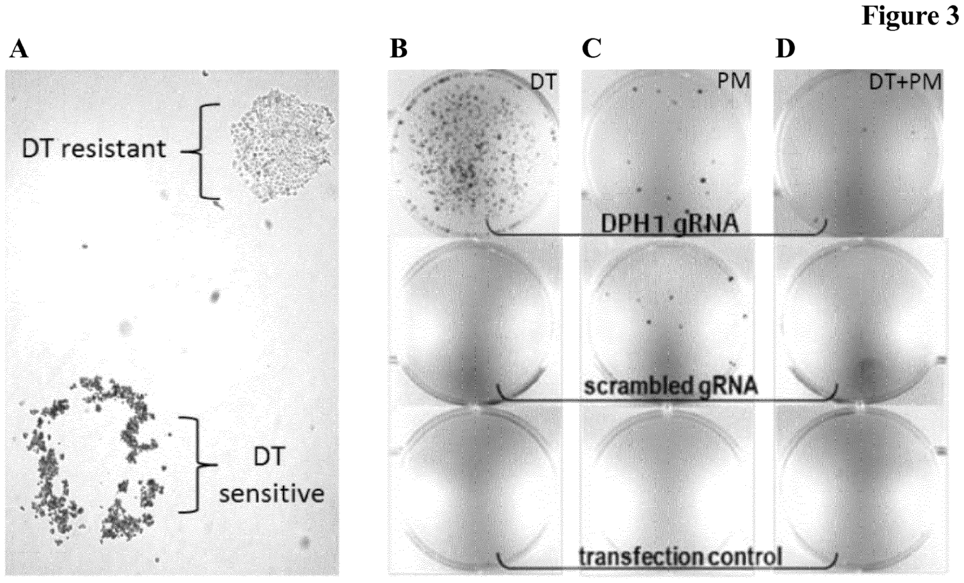

Diphtheria toxin (DT) ADP-ribosylates diphthamide and thereby inactivates eukaryotic translation elongation factor 2 (eEF2). This irreversibly stalls protein synthesis and kills cells (Weidle et al.). Diphthamide is a histidine modification placed upon eEF2 by diphthamide synthesis gene encoded enzymes, among them DPH1. Complete inactivation of DPH1 in MCF7 cells prevents the synthesis of the toxin target diphthamide. This renders cells resistant to DT (Stahl et al.). In consequence, inactivation of all copies of DPH1 generates the phenotype 'DT-resistance' (DTr). The frequency of this phenotype can be detected in a robust manner by counting toxin resistant colonies (see Figure 3).

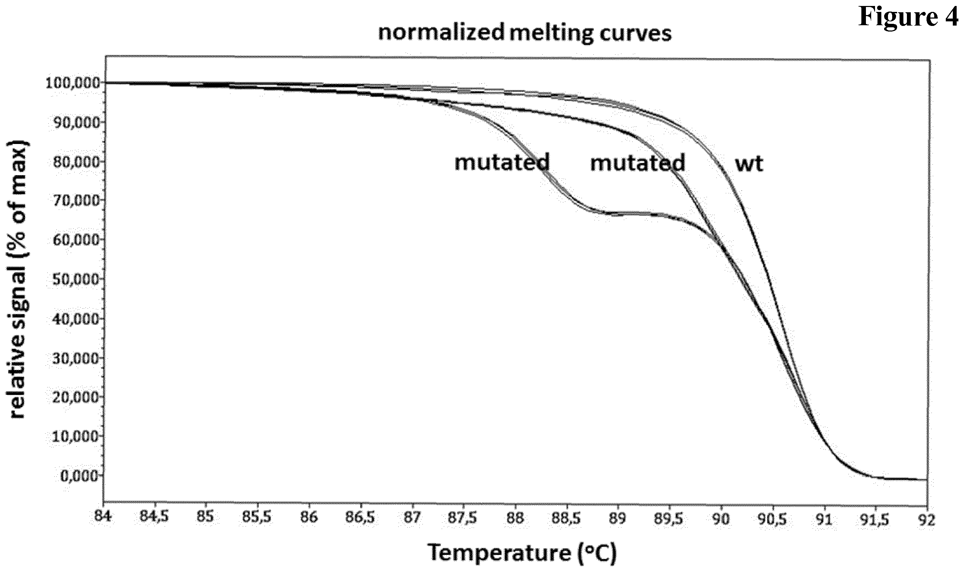

Because presence of one remaining functional DPH1 allele is sufficient for toxin sensitivity, DTr cells harbor functional knockouts of all DPH1 alleles. Cells which have only one allele modified can be identified by on-cell HRM-PCR assays (Figure 4). Modification of the target site alters the melting temperature of a DPH1-PCR fragment compared to that of the wild-type fragment, generating a bi-phasic HRM-profile. As CRISPR-Cas9 mediated gene-inactivations are independent events and hence rarely identical on both alleles, most cells with complete gene inactivation will also show bi-phasic HRM-profiles. These can be differentiated from mono-allelic alterations by their toxin resistant phenotype.

Thus, as in one embodiment a combination of (high throughput) HRM-PCR and DTr colony count assays quantifies and differentiates heterozygous and homozygous inactivation events.

### Selection marker resistance detects and differentiates specific and non-specific integration events

Puromycin-N-acetyl-transferase (PAC), encoded by the integration cassette of the CRISPR/Cas9 donor plasmid used in the current example, inactivates puromycin (PM) and renders modified cells PM-resistant (Vara et al.). Thus, PAC integration becomes detected and quantified by PM-resistance assays in the same manner as described for DTr colonies (applying PM instead of DT as selection agent, Figure 3B). In contrast to DTr, which results only from specific and homozygous target gene inactivation, PMr occurs independent from the position of integration. The frequencies of site specific versus non-(site-)specific integration are, as in one embodiment, addressed by comparing double resistant colonies (DTr+PMr) and PMr colonies.

### Comparing CRISPR/Cas9 inflicted DPH1 inactivation and targeted integration events

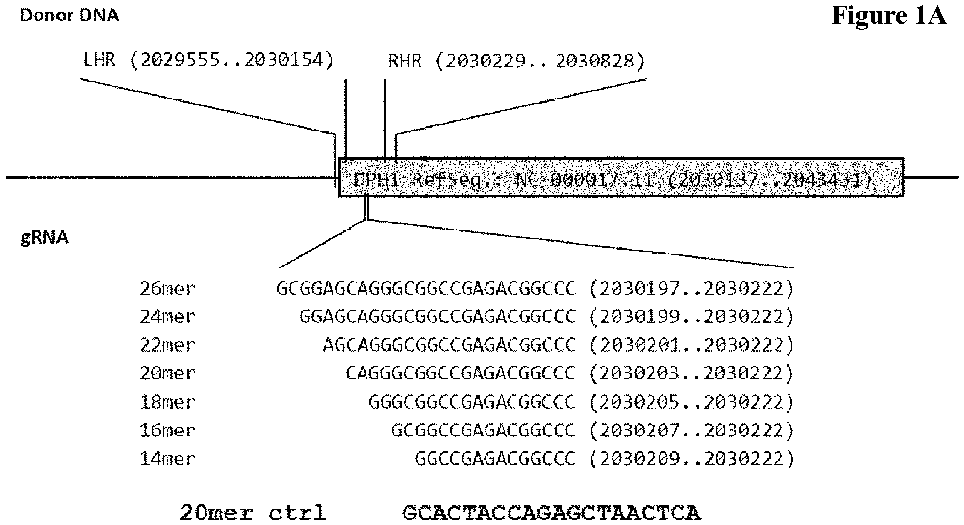

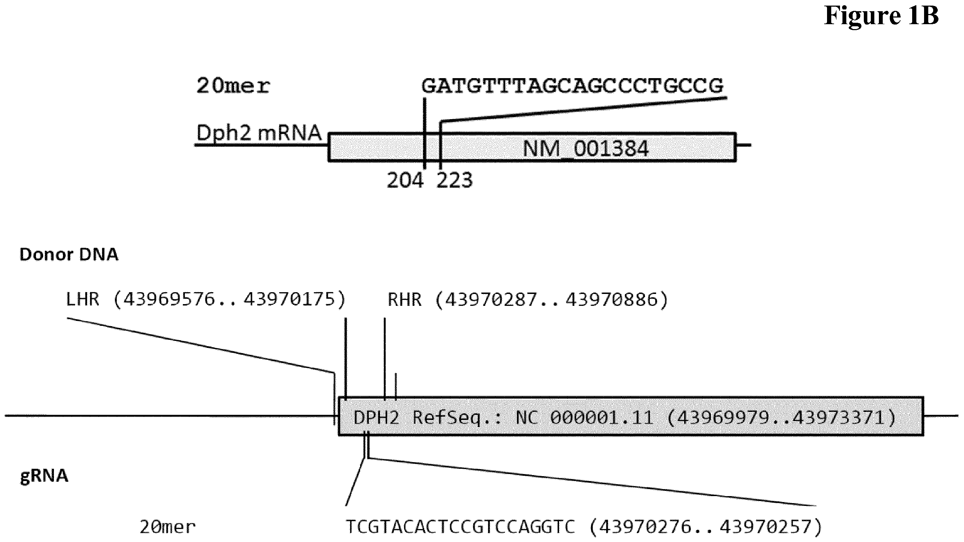

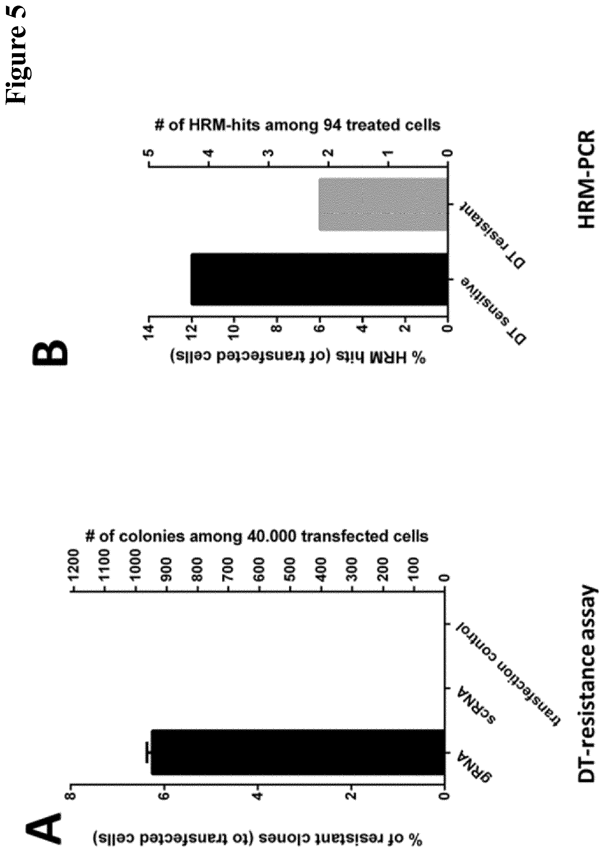

To compare the frequencies of target-specific inactivation, integration and non-target integration, plasmids encoding DPH1 specific CRISPR/Cas9 modules (see Figure 1 for sequences) were transfected into MCF7 cells. These were subsequently subjected to HRM-PCR as well as to colony count assays that detect DT- and PM resistances. The results of these assays are summarized in Figure 5, individual data sets are shown in the following Tables. Figure 5A shows that complete inactivation of the DPH1 gene indicating functional loss of all DPH1 alleles occurred with a frequency of ~6% of all transfected cells (2.5% of all cells considering transfection efficiencies of 40%, see first table below). DPH1 inactivation showed absolute dependency on matching gRNA sequence: scrambled control RNA (scRNA) did not generate any DTr colony. A comparison of the frequencies of HRM identified clones with occurrence of DTr colonies is shown in Figure 5B. These analyses revealed that mono-allelic gene inactivation (toxin sensitive HRM confirmed) occurred twice as frequent as inactivation of both alleles (DTr cells).

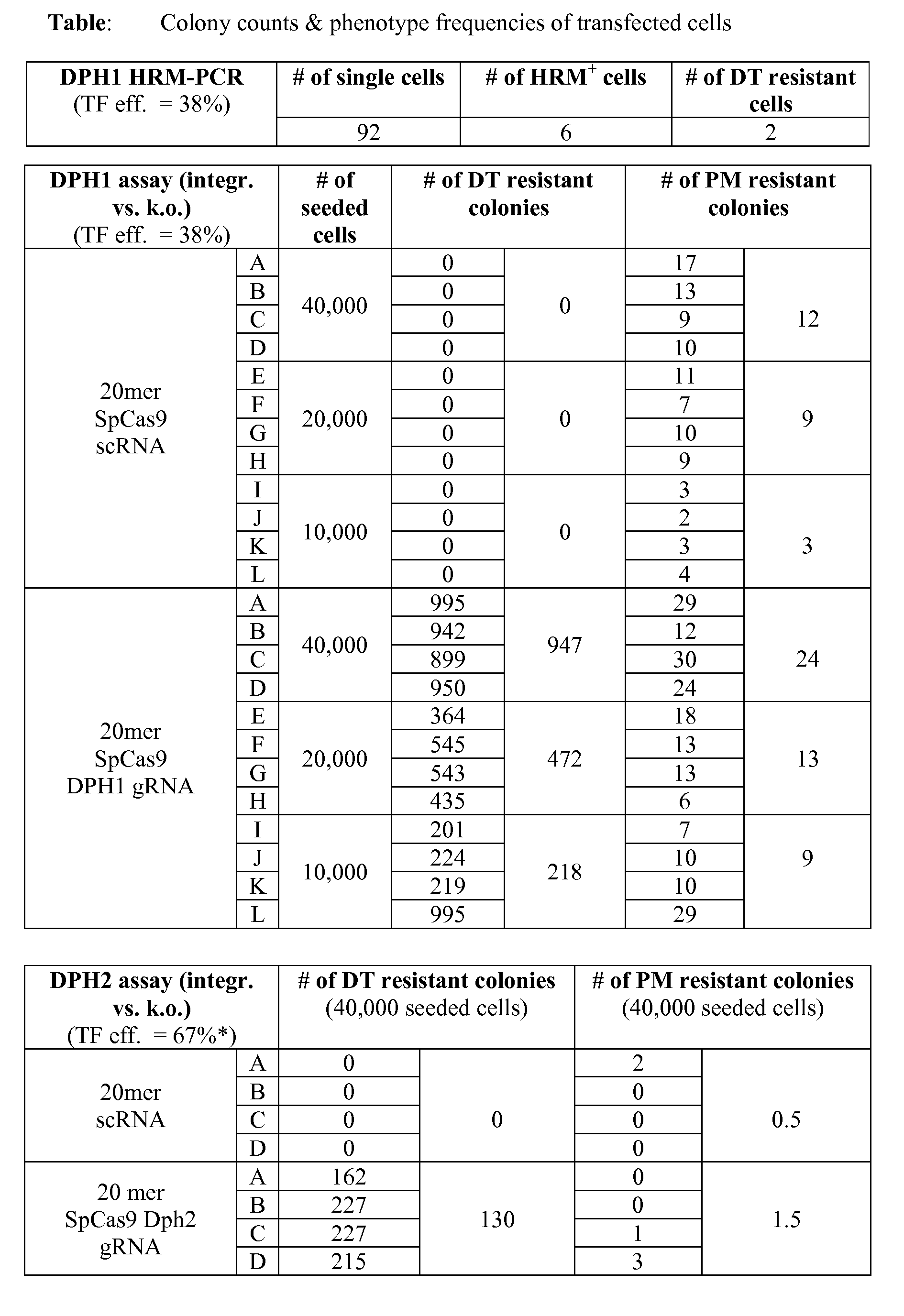

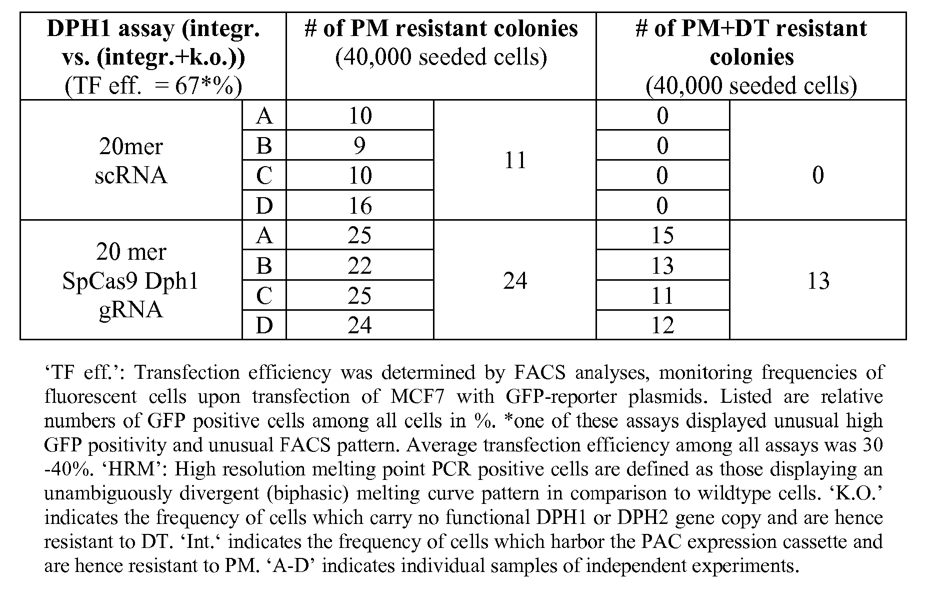

**Table: Influence of gRNA length on targeted gene inactivation and cassette integration**

| **DPH1 (gRNA length)** (TF eff. = 31%) | | # **of DT resistant colonies** (20,000 seeded cells) | | **# of PM resistant colonies** (40,000 seeded cells) | | # **of DT+PM resistant colonies** (40,000 seeded cells) | |
|---|---|---|---|---|---|---|---|
| 20mer scRNA | A | 0 | 0 | 7 | 8 | 0 | 0 |
| | B | 0 | | 6 | | 0 | |
| | c | 0 | | 9 | | 0 | |
| | D | 0 | | 10 | | 0 | |
| 14 mer SpCas9 Dphl gRNA | A | 0 | 0 | 6 | 8 | 0 | 0 |
| | B | 0 | | 10 | | 0 | |
| | c | 0 | | 8 | | 0 | |
| | D | 0 | | 9 | | 0 | |
| 16 mer SpCas9 Dphl gRNA | A | 275 | 284 | 21 | 25 | 7 | 8 |
| | B | 298 | | 26 | | 9 | |
| | c | 292 | | 25 | | 10 | |
| | D | 271 | | 26 | | 9 | |
| 18 mer SpCas9 Dphl gRNA | A | 284 | 307 | 19 | 23 | 10 | 10 |
| | B | 299 | | 25 | | 12 | |
| | c | 321 | | 27 | | 7 | |
| | D | 324 | | 22 | | 11 | |
| 20 mer SpCas9 Dphl gRNA | A | 417 | 409 | 16 | 15 | 6 | 6 |
| | B | 406 | | 20 | | 5 | |
| | c | 409 | | 16 | | 7 | |
| | D | 404 | | 8 | | 7 | |

| **DPH1 (gRNA length)** (TF eff. =31%) | | **# of DT resistant colonies** (20,000 seeded cells) | | **# of PM resistant colonies** (40,000 seeded cells) | | **# of DT+PM resistant colonies** (40,000 seeded cells) | |
|---|---|---|---|---|---|---|---|
| 22 mer SpCas9 Dphl gRNA | A | 278 | 284 | 14 | 13 | 6 | 5 |
| | B | 292 | | 12 | | 5 | |
| | c | 297 | | 11 | | 4 | |
| | D | 267 | | 15 | | 5 | |
| 24 mer SpCas9 Dphl gRNA | A | 237 | 232 | 13 | 13 | 2 | 3 |
| | B | 252 | | 12 | | 4 | |
| | c | 228 | | 11 | | 3 | |
| | D | 211 | | 15 | | 3 | |
| 26 mer SpCas9 Dphl gRNA | A | 161 | 170 | 12 | 11 | 0 | 1 |
| | B | 155 | | 11 | | 1 | |
| | c | 176 | | 12 | | 0 | |
| | D | 187 | | 9 | | 1 | |

| | | | | | | | |
|---|---|---|---|---|---|---|---|
| 'TF eff.': Transfection efficiency was determined by FACS analyses, monitoring frequencies of fluorescent cells upon transfection of MCF7 with GFP-reporter plasmids. Listed are relative numbers of GFP positive cells among all cells in %. Cells which carry no functional DPH1 gene copy are resistant to DT. Cells which harbor the PAC expression cassette are hence resistant to PM. 'A-D' indicates individual samples of independent (quadruplicate) experiments. | | | | | | | |

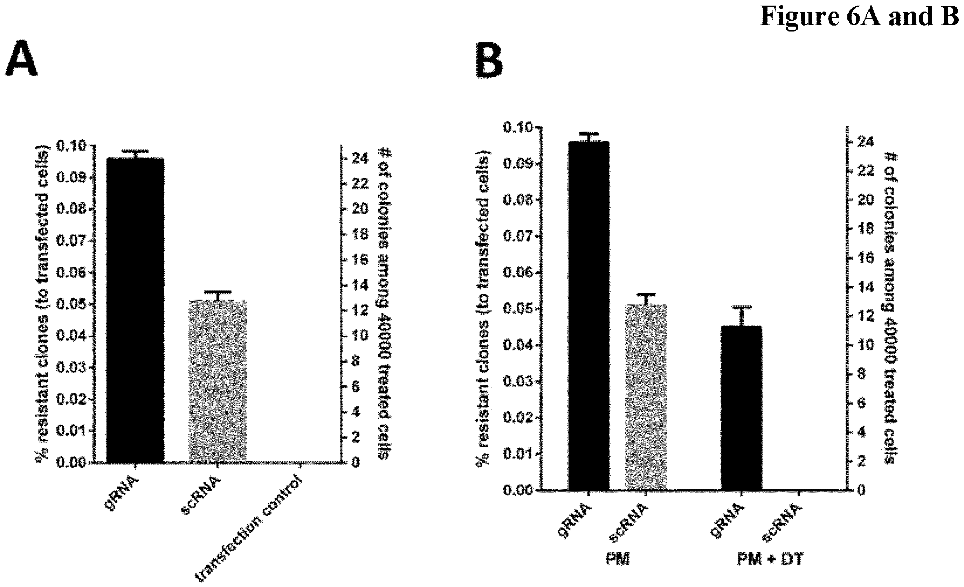

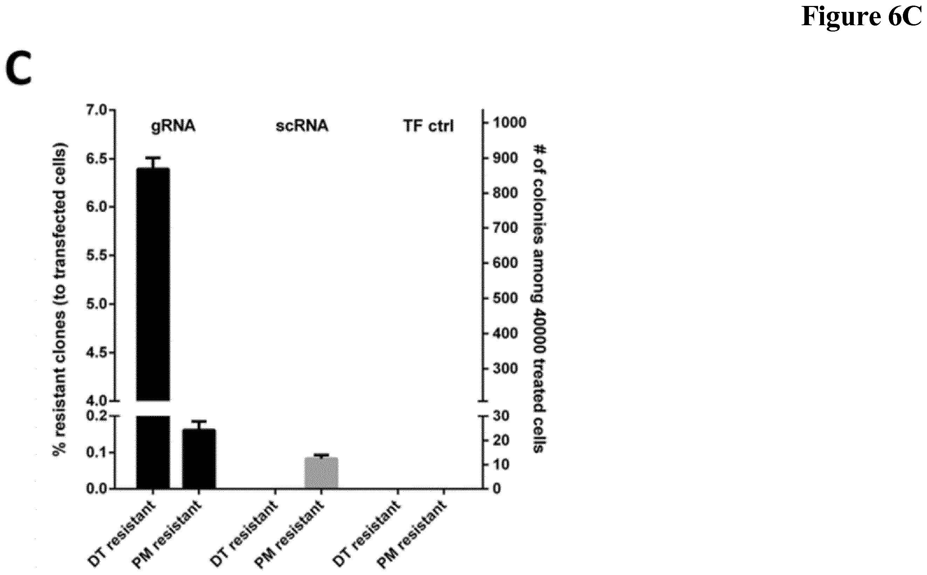

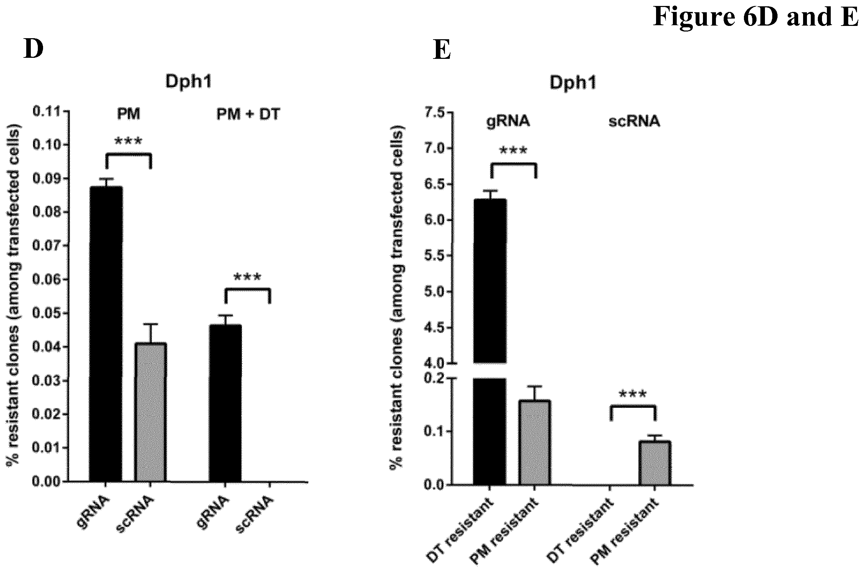

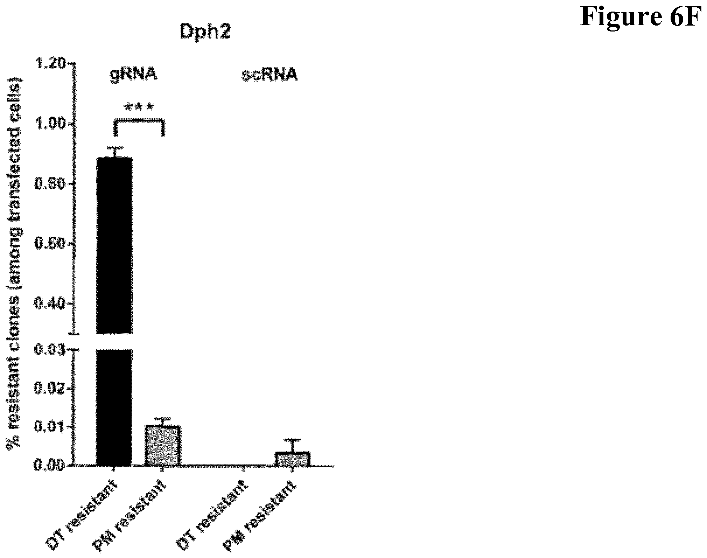

Figure 6 shows a comparison of the frequencies of DTr and PMr colonies (two different experiments, experiment 1: A-C, experiment 2: D-F): inactivation of both DPH1 alleles occurred with 30-50 fold higher efficacy than integration of the PM-resistance mediating PAC expression cassette (Figures 6C and 6E, position or zygosity of PAC integration cannot be determined). Compared to DPH1-specific gRNA, scRNA generated under otherwise identical conditions 2-fold less PMr colonies. Thus, Cas9/DPH1-gRNA mediated integration occurs with preference at the DPH1 gene, but not with absolute specificity. Thus, such effect can be confirmed with the method as reported herein. In accordance with preferential integration in the DPH1 gene, many PMr colonies obtained with the DPH1 guide RNA were DT resistant (Figures 6A and 6D). None of the PMr colonies obtained with scRNA were resistant to DT. Thus, Cas9 mediated gene inactivation (incl. on both alleles) occurs highly specific and with much higher frequency than targeted PAC integration (Figures 6C and 6E). Thus, such effect can be confirmed with the method as reported herein. Targeted integration occurs not only less frequent but is also less specific for the position defined by the gRNA.

### Quantification of gene editing works in an equal manner with another target gene, DPH2

The identical approach as outlined above was used also with a different gene, the DPH2 gene. That is, Cas9-induced modification of the DPH2 gene was performed. This was done to show the general applicability of the method as reported herein.

DPH2 encodes a different enzyme with a different sequence on a different chromosome, yet is also essential for diphthamide synthesis. DPH2 deficiency renders cells resistant to DT in the same manner as DPH1 deficiency (Stahl et al.).

The results of DPH2 editing followed by assessment of DT- and PM-resistances are shown in Figure 6F: in line with our observations on DPH1, homozygous DPH2 inactivation events were observed with higher frequencies than integration of the PAC expression cassette with a change within the same order of magnitude (~90 fold higher inactivation of DPH2 than integration of the PAC expression cassette). Inactivation was strictly dependent on presence of cognate gRNA while cassette integration had site-preference but not absolute specificity for the target gene (comparing frequencies of DPH2 guide with scRNA).

Thus, the assay principles developed to characterize DPH1 modification can also be applied to analyze DPH2 modification.

The result with respect to the use of two different genes, DPH1 and DPH2, provide proof that this assay system is transferable to other genes.

### Comparison and optimization of Cas9 gene manipulation modules - gRNA length

As shown above, the method as reported herein can address general effects of the composition of gene modifying modules.

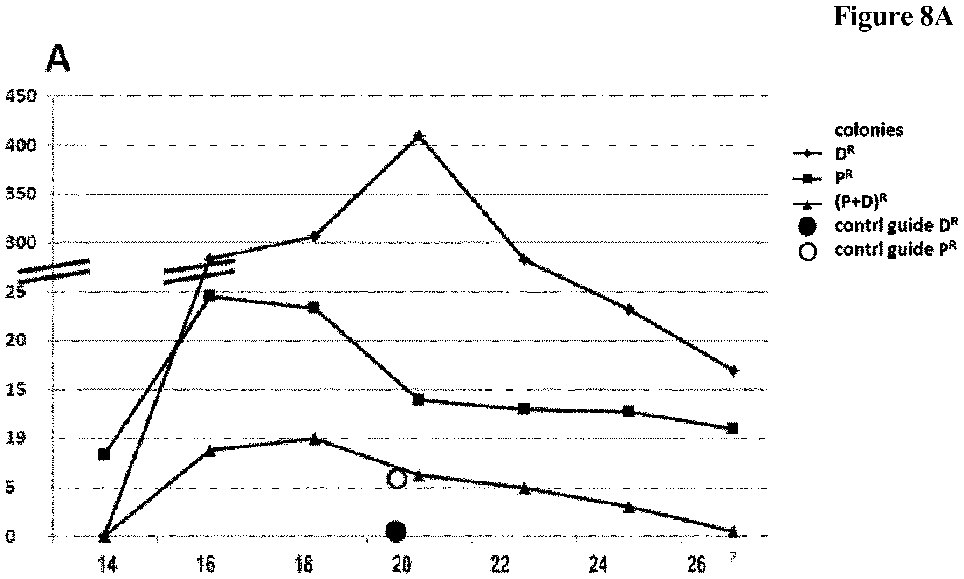

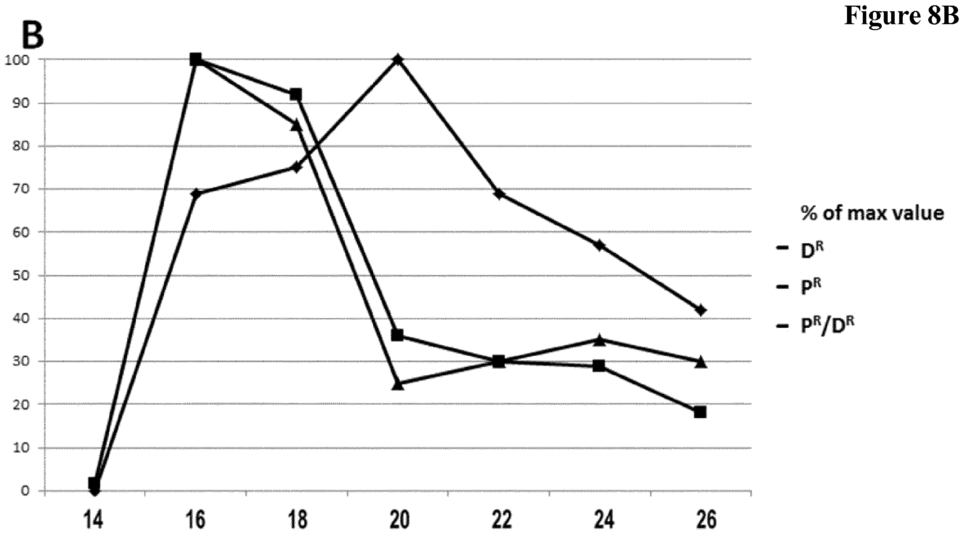

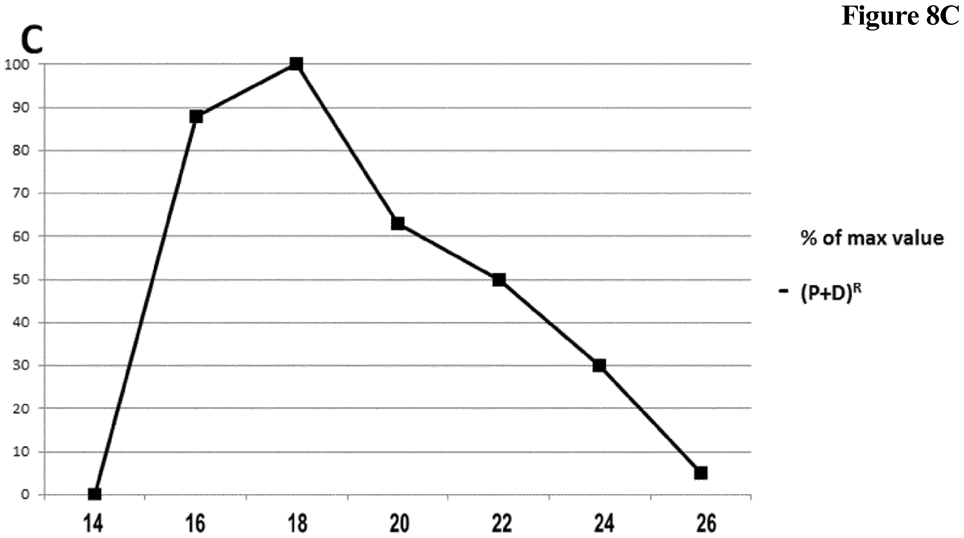

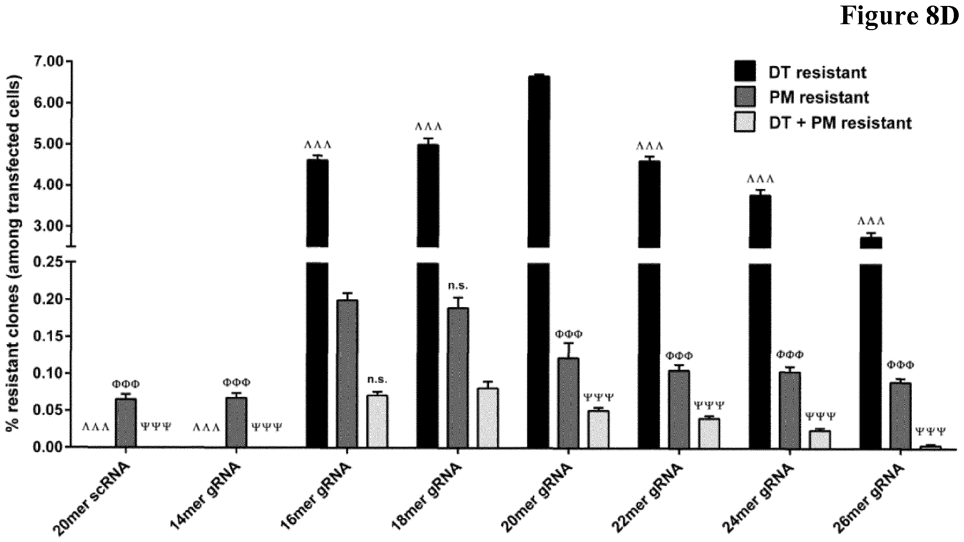

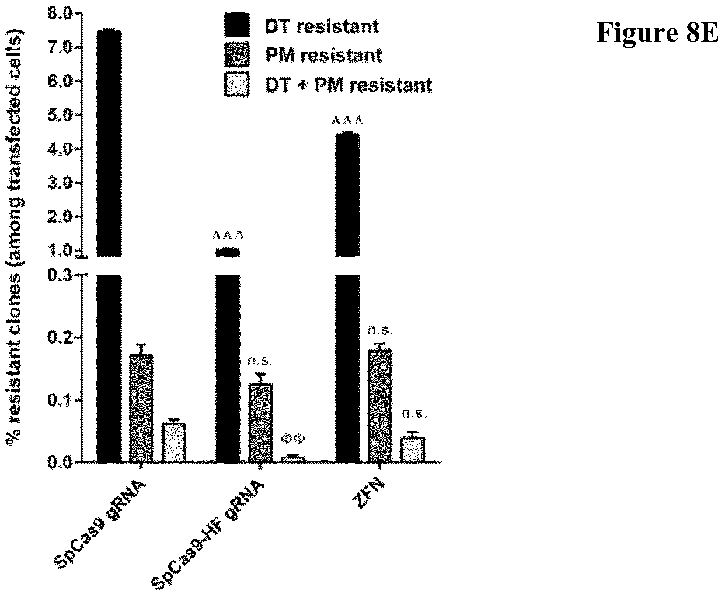

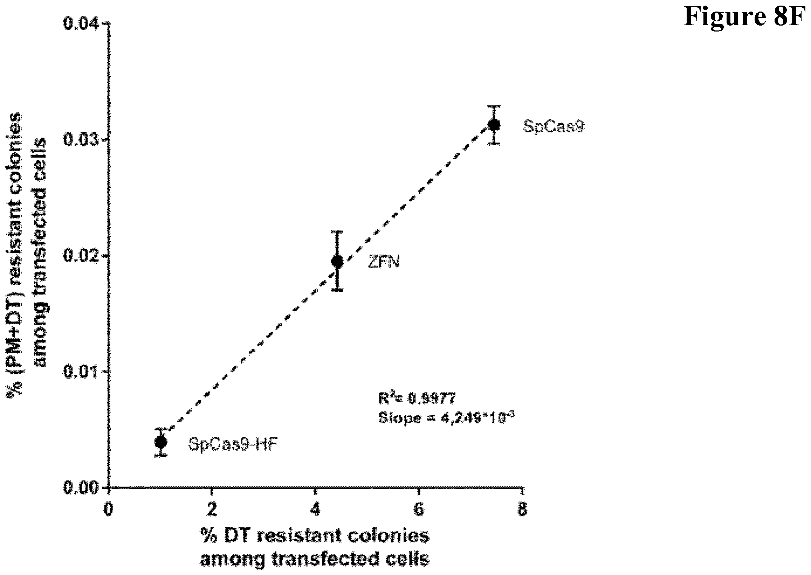

Figure 8 shows how gene inactivation as well as integration efficacy and specificity of Cas9 gRNAs of different lengths can be assessed and compared. All applied gRNAs targeted the same sequence stretch within DPH1 but varied in lengths from 14 to 26 bases (Figure 8D, gRNA details in Figure 1). DTr colony numbers were recorded to reflect target gene specific homozygous inactivation. Simultaneously, numbers of PMr and of DTr-PMr double resistances were assessed to monitor cassette integration.

It can be shown with the method as reported herein that gRNA length influences the efficacy of gene inactivation.

It was shown that 20mers confer maximum DPH1 inactivation efficacy. By shortening the complementary stretch to 18 or 16 bases or extending it up to 26 bases it was determined with the method as reported herein that significant specific gene inactivation functionality was retained, albeit with decreased efficacy than the 20mer.

Reducing the gRNA to less than 16 bases (14mer) decreased DPH1 inactivating functionality to below detection levels. Integration efficacy (assessed by counting PMr events) was also influenced by gRNA lengths. Guide RNAs of less than 16mers (14 mer) generated only few PMr colonies, not exceeding scrambled control background levels. Targeted integration was observed for 16mers, 18mers, 20mers, 22mers, 24mers and 26mers, reaching an optimum of insertion efficacy with 16-18mer.

It was thus shown with the method as reported herein that no efficacy gain was achieved with larger oligonucleotides, in fact larger size reduced the specific insertion events significantly.

The ratio between integration events (PMr) and inactivation events (DTr) can be calculated as a 'specificity indicator' to identify conditions at which specific integration occurs with the least gene inactivation events using the method as reported.

Such conditions may be favored if one desires targeted integration without inflicting excessive non-productive target gene damage. Low values (e.g. few PMr relative to DTr colonies) reflect inefficient integration in relation to simultaneous occurring inactivation events. High values (more PMr and/or relatively decreased numbers of DTr colonies) reflect more efficient integration at Cas9-targeted genes.

Figure 8E shows the calculated specificity factors in dependence on lengths of gRNA. It was shown with the method as reported herein that highest insertion per inactivation values are found for 16-18mers and a clear drop for guide RNAs containing 20 bases or more. This shows that 20mers are quite efficient for targeted gene inactivation (in agreement with previously published observations, see e.g. Cho et al. 2013, Bauer et al., Jinek et al., 2012 and 2013, Mali et al. 2013). Yet shorter guide RNAs appear to be more efficient for integration. Shorter guide RNAs improve not only the integration efficacy (higher overall numbers of PMr colonies), but also the ratio between productive and non-productive gene editing (less DTr colonies without insertion).

### Efficacy and specificity of different gene editing approaches - enzymes

The method as reported herein was used to compare gene inactivation and integration events, efficacy and specificity of different variants of RNA-guided Cas9 as well as ZFN-mediated gene editing.

Therefore, gRNA length and composition was kept constant (DPH1 20mer), three different editing enzymes were applied:
(i) 'SpCAS9' specifies the Cas9 nuclease from Streptococcus pyogenes, which can be considered as current standard application (see e.g. Ran et al. 2013, Fu et al. 2014);
(ii) SpCas9-HFl is an engineered variant of SpCas9 with reduced unspecific DNA binding, reduced off-target activity, and hence with proposed higher fidelity & specificity (Kleinstiver et al.);
(iii) a ZFN-mediated editing module which recognizes target sequences by designed zinc finger mediated protein-nucleic acid interactions (Miller et al. 2011, Urnov et al.).

In the same manner as for gRNA analyses, DTr colonies were recorded to reflect targeted gene inactivation, PMr colonies to monitor cassette integration (Figure 8F; Table below).

**Table: Colony counts and phenotype frequencies of MCF-7 cells transfected with different gene editing entities**

| **DPH1 assay editing entities** (TF eff. = 33%) | # **of DT resistant colonies** (40,000 seeded cells) | # **of PM resistant colonies** (80,000 seeded cells) | **# of DT+PM resistant colonies** (80,000 seeded cells) |
|---|---|---|---|
| SpCas9 scRNA | 0 | 4 | 0 |
| | 0 | 3 | 0 |
| | 0 | 5 | 0 |
| | 0 | 4 | 0 |
| SpCas9 Dphl gRNA | 476 | 14 | 5 |
| | 492 | 11 | 4 |
| | 468 | 9 | 4 |
| | 472 | 10 | 3 |
| SpCas9-HF scRNA | 0 | 2 | 0 |
| | 0 | 1 | 0 |
| | 0 | 0 | 0 |
| | 0 | 0 | 0 |
| SpCas9-HF Dphl gRNA | 60 | 9 | 1 |
| | 70 | 10 | 1 |
| | 66 | 8 | 0 |
| | 62 | 5 | 0 |
| ZFN-Dph1 | 286 | 12 | 4 |
| | 292 | 13 | 1 |
| | 276 | 10 | 2 |
| | 278 | 11 | 3 |

| | | | |
|---|---|---|---|
| MCF-7 cells were transfected with plasmids encoding different genome editing systems (SpCas9, SpCas9, ZFN). The SpCas9 construct was as described before. SpCas9-HF includes the N497A/R661A/Q695A/Q926A substitutions according to Kleinstiver et al. (Kleinstiver et al. 2016). In parallel, gRNAs were replaced by scRNAs in parallel to address non-specific activity. DPH1-specific ZFN was obtained from Sigma Aldrich (CompoZr^{®}). The total amount of plasmid DNA (editing entity and donor) for transfection of the initial cell pool of 3^{∗}106 cells was as described for the previous experiments. To quantify the transfection efficiency (TF eff. (%)), GFP-reporter plasmids were transfected aside. GFP-positive cells were counted 24h after transfection by FACS. Defined numbers of cells were seeded (#seeded cells) and treated with DT, PM, or DT+PM 72 hours thereafter. | | | |

Comparing overall efficiencies of gene inactivation and cassette integration determined with the method as reported herein the following was found:
Highest values for gene inactivation and cassette integration were determined for CRISPR/SpCas9.
CRISPR/SpCas9-HF diminished targeted gene inactivation events to less than 20% of the number of DTr colonies compared to CRISPR/SpCas9. Frequencies of PMr (integration) and DT-PM double resistant colonies (integration with targeted gene inactivation) were also reduced.
ZFN modules reduced the number of DTr colonies under otherwise identical conditions to less than 60% of the events observed with CRISPR/SpCas9. The efficacy of ZFN-targeted inactivation was thus ~2-fold reduced compared to SpCAS9 and ~2-3 fold better than that of the engineered SpCas9-HF1. PMr colony frequency did not significantly differ between CRISPR/SpCas9 and ZFN. Double resistant colonies (cassette integration with simultaneous gene inactivation) were somewhat (30%) reduced with ZFN compared to CRISPR/SpCas9.

Calculation of the ratio of DTr (target gene inactivation) to DT+PM double-resistant (target site integration) colonies takes overall efficacy out of the equation: CRISPR/SpCas9, CRISPR/Cas9-HF, as well as ZFN generated the same level (~4×10⁻³) of targeted integration events per one homozygous gene inactivation event.

Thus, by using the method as reported herein it could be shown that overall efficacies of editing systems vary, but 'specificities' of cassette integration above the background of target gene inactivation appear to be not significantly different for all approaches.

### Influence of DNA repair modulators on gene editing efficacy and specificity

The method as reported herein (comprising colony assays to determine DTr and PMr cells upon DPH gene editing) can also be used to address the influence of compounds that modulate DNA repair.

Activators of homologous recombination (HR) and inhibitors of non-homologous end joining (NHEJ) are described to modulate gene editing events and increase integration efficacies (Song et al., Ma et al.).

In order to demonstrate the usefulness of the method as reported herein for determining the effect of DNA repair modulators on editing efficacy and specificity, CRISPR/SpCas9/DPHIgRNA(20mer) editing assays performed according to the method as reported herein were supplemented with such compounds and the influence quantified.

The DNA ligase IV inhibitor SCR7 was applied either 4hrs before transfection ('early addition') or 18hrs after transfection ('late addition') of the gene editing modules, continuing exposure until 72hrs after transfection. In the same manner, the RAD51 modulator RS-1 (RAD51-stimulatory compound 1) was added to stimulate HR. Both compounds were applied at doses that had no effect on the growth or viability of MCF7 cells: 1 µM for Scr7 and 8 µM for RS-1, and 1 µM + 8 µM (SCR7+RS1) when combining both. Compared to the untreated control (no compound) addition of RS-1 was determined to increase the number of PMr colonies ~2fold without affecting the number of colonies obtained with scRNA (see Table below).

**Table: Phenotypes of MCF-7 exposed to SCR7 and/or RS-1 during gene editing**

| **compound** | **compound addition** | **mean DTr colonies #seeded cells: 40,000** | **mean PMr colonies #seeded cells: 80,000** | **% PMr relative to DTr colonies** |
|---|---|---|---|---|
| none | 4hrs before transfection | 200.5 (213;203;194;192) | 8.5 (6;11;8;9) | 4.2 % |
| | 18hrs after transfection | 515 (522;513;507;518) | 20 (20;24;17;19) | 3.9% |
| RS-1 (8 µM) | 4hrs before transfection | 201.5 (196;202;210;198) | 14 (11;13;15;17) | 6.9 %^{∗} |
| | 18hrs after transfection | 512.5 (512;521;506;511) | 15.3 (17;15;14;15) | 3.0% |
| SCR7 (1 µM) | 4hrs before transfection | 205.3 (215;193;205;208) | 13.3 (11;11;16;15) | 6.5%^{∗} |
| | 18hrs after transfection | 488.8 (486;482;491;496) | 25 (26;25;27;22) | 5.1 % |
| RS-1+SCR7 (8 µM + 1 µM) | 4hrs before transfection | 175 (183;175;177;165) | 14.3 (12;14;15;16) | 8.1 %^{∗∗∗} |
| | 18hrs after transfection | 488.3 (492;485;495;481) | 10.25 (8;10;6;17) | 2.1%^{∗∗} |

| | | | | |
|---|---|---|---|---|
| Cells transfected with plasmids for SpCas9-mediated DPH1 editing were seeded in defined numbers (#seeded cells). DT/PM selection started 72hr after transfection. Values (w,x,y,z) indicate colonies obtained in quadruplicate individual experiments. Influence of the time point of the compounds (RS-1, SCR7 and RS-1+SCR7) addition was. Significant difference of PM resistant relative to DT resistant colonies of treated samples vs. no compound is indicated with ^{∗}p<0.05, ^{∗∗}p<0.01, ^{∗∗∗}p<0.001. | | | | |

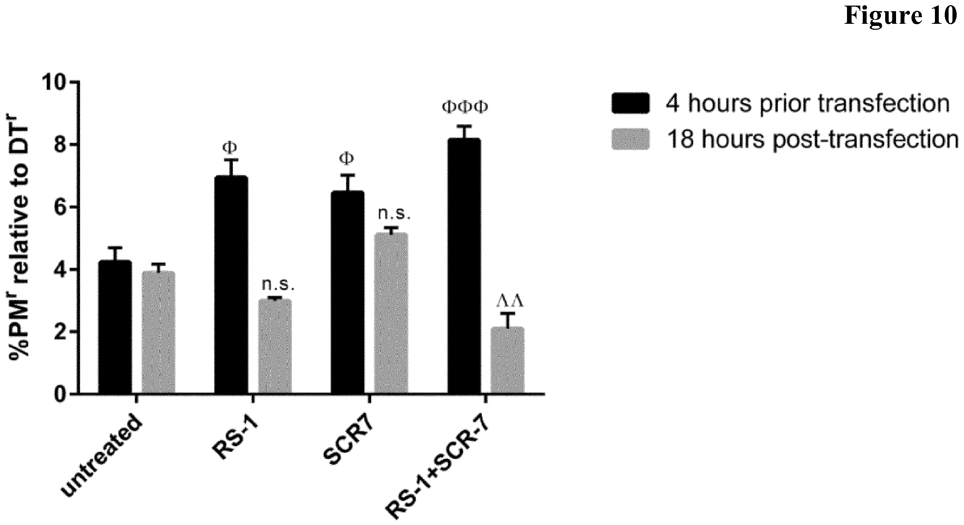

To quantify the effect on the overall integration efficacy the percentage of PMr colonies (gene integration) relative to DTr colonies (gene inactivation) was determined with the method as reported herein (see also Figure 10).

It has been found based on the results of the method as reported herein that the addition of RS-1 at an early time point lead to a significantly higher integration efficiency, however it did not affect integration efficiency upon late addition (18hrs after initiation of editing). Thus, choosing the appropriate (early) time point for RS1-mediated HR stimulation is important for productive editing. This confirms HR to be a driver for targeted cassette integration.

It has also been found based on the results of the method as reported herein that to a similar degree early application of SCR7 significantly increased the relative number of integration (Figure 10 & Table above). This confirms previous observations of enhanced productive gene editing upon SCR7 administration (see Ma et al.).

It has been further found based on the results of the method as reported herein that combining both compounds the PMr relative to DTr ratio was 8.1% compared to 6.5% (only SCR7) or 6.9% (only RS-1). This difference/increase was however without significance (p=0.39 vs RS-1 alone) which is in line with previous observations (Pinder et al., Gutscher et al.).

### Application of high throughput technologies to identify conditions or components or condition-component combinations that enable improved gene editing

The method as reported herein is based on the editing-based inactivation of cellular genes that confer sensitivity towards lethal eEF2-inactivating toxins such as DT or Pseudomonas Exotoxin A or Cholix toxin. The read-out of these assays is 'colony-count' of cells resistant to such an ADP-ribosylating toxin.

Death vs. survival-readouts are very robust and cell based colony count approaches are high-throughput compatible. Therefore, the assay principle can be adapted to highly parallel assay set-up and run on automated (robotics) platforms. Automated high-throughput platforms for phenotype-identification in cell based assays are available in various formats and are well known in the field.

High throughput/automated application of the described DPH-editing based assay principle measures the influence of large numbers of different conditions or parameters or additives/modulators on gene editing, in particular on efficiency and specificity. Conditions or parameters or additives/modulators are addressed in including (exemplarily without imposing a limitation to these) collections or libraries of synthetic or natural compounds. This identifies compounds and conditions that enhance efficiency or specificity of gene editing. Such compounds and conditions are in particular derived from or similar to in structure or function to entities and compound classes that directly or indirectly influence DNA recombination and repair, nucleic acid binding, interaction of proteins with nucleic acids or interaction with proteins that are involved in nucleic acid rearrangement, metabolism, synthesis or repair.

### Outlook

Genome editing has emerged as technology of utmost importance for scientific applications. Its entire potential, however, is still limited by efficacy and specificity issues of currently applied editing approaches. The herein reported method enables simple and robust quantification and comparison of efficacy and specificity of editing-inflicted gene inactivation and insertion. It allows exact determination of heterozygous and homozygous gene inactivation and non-specific versus targeted integration events, based on large numbers of individual cells. Learnings and parameters generated by this method can improve not only the science of gene editing, but may be of particular importance for developing and optimizing gene editing.

The core principle of the method as reported herein comprises editing-based inactivation of cellular genes that confer sensitivity towards lethal eEF2-inactivating toxins such as DT. The toxin target is a diphthamide placed on eEF2 by DPH-gene encoded proteins (e.g. DPH1 or DPH2). Complete loss of functionality (both alleles) of either of these genes results in 'absolute' toxin resistance as it prevents diphthamide synthesis. Thus toxin resistance specifically indicates homozygous functional inactivation of both target genes. Cells which have only one target allele inactivated are still toxin sensitive. Integration events can be separately assessed by resistance conferring genes on integration cassettes. Toxin resistance via inactivation of DPH genes is 'absolute', without any background and no matter how much toxin is applied. This is a key factor for robustness and simplicity of the method: frequencies of homozygous target gene inactivation events can be assessed by counting toxin resistant colonies, insertion events by PM-resistant colonies and double events by counting colonies that are resistant to both. In consequence the method enables a simple and robust assessment of individual editing events on large numbers of individual cells. Furthermore (and in contrast to many existing tools, see e.g. Fu et al. 2014, Doench et al., Maruyama et al., Shalem, et al.) homozygous and heterozygous gene inactivation and integration events can be differentiated. Thus, simple colony counts reflect efficacies of and ratios between productive (integration) and destructive gene editing (inactivation without integration).

The method delivers 'generalizable' results was evidenced by the results of comparing editing events (colony frequencies) on two different DPH genes. DPH1 and DPH2 encode different enzymes, both of which are independently essential for diphthamide synthesis. The results showed comparable efficacies, specificities and destruction/integration ratios for both genes. Comparable readouts despite of different target sequences on different genes on different chromosomes shows that the rules, dependencies and parameters determined by applying this method are transferrable to optimize editing of other genes.

The application of DPH gene inactivation as a robust method to characterize, differentiate and optimize gene editing efficiency is not restricted to CRISPR/Cas modules. It can be applied in the same manner with identical readout parameters to other gene editing principles such as zinc finger nucleases or TALENS (Carroll 2011; Christian, et al. 2010; Gaj, et al. 2013; Miller, et al. 2011; Urnov, et al. 2010). The observation of similar overall gene inactivation and integration frequencies on different DPH target genes (located on different chromosomes) indicates also that the 'rules', dependencies and optimization parameters derived from this approach are generalizable (i.e. transferrable to other genes). Thus, gene editing modules and parameters can be optimized by DPH gene modification and thereafter transferred to optimize gene editing efficiency or specificity of other genes.

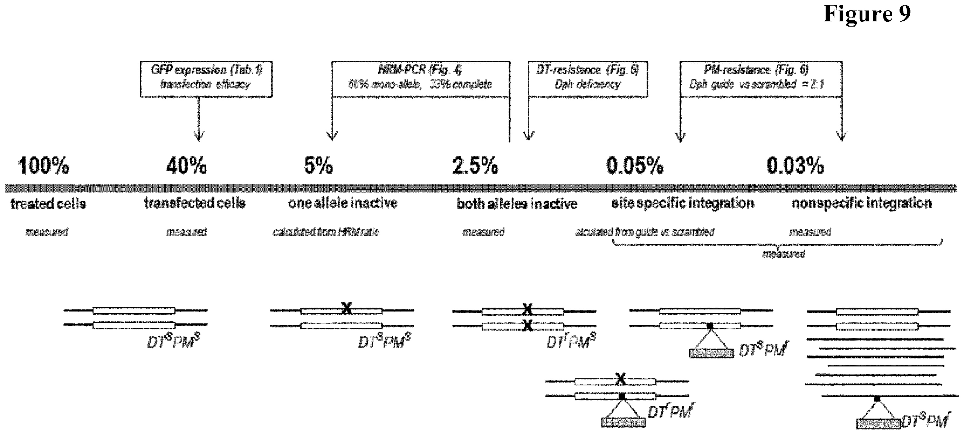

With the method as reported herein previous observations ((Cho, et al. 2013; Jinek, et al. 2012; Jinek, et al. 2013; Mali, et al. 2013b) that 20mer guide RNAs are effective for CRISPR/Cas mediated gene targeting could be re-confirmed. Highest overall gene inactivation and integration frequencies were observed with 20mers. In contrast to many other evaluation methods (Doench, et al. 2014; Fu, et al. 2014; Maruyama, et al. 2015; Shalem, et al. 2014), in the method as reported herein not only overall frequencies are assessed, but also homozygous and heterozygous gene inactivation (and non-(site-)specific integration) events can be differentiated in a rapid, simple and robust manner on a large number of individual cells (see Figure 9). This enabled to compare the frequencies of and ratios between productive gene editing (cassette integration) and destructive gene editing events (gene inactivation without integration).

These analyses lead to the interesting observation that (albeit highest overall inactivation frequencies were achieved with 20mers), the 'best' ratio between productive and destructive was observed with 16-18mer guide RNAs. Thus, 20mers, which can still be considered as current gRNA 'standard' (Haeussler, et al. 2016; Liao, et al. 2015; Muller, et al. 2016) may be the choice if one aims for most efficient gene inactivation. On the other hand, the results presented herein indicate that 16-18mers may be preferred for editing events if one desires integration without excessive destructive editing.

It is interesting to note that Fu et al., (Fu, et al. 2014), have evaluated gRNAs sized shorter than 20mers in gene inactivation experiments and observed inactivation efficacies comparable to 20mers with reduced off-target effects. Their analyses were based upon mono-allelic GFP gene inactivation and in consequence (just one target gene per cell) could not address or differentiate homozygous and heterozygous inactivation events in diploid cells. Fu et al. could also not quantify and compare insertion events. The data (based on large numbers of cells and on inactivation of normal chromosome encoded human genes) indicate that 20mers are more efficient inducers of gene inactivation than shorter guides. Shorter guides, however, were more efficient mediators of insertions.

Optimal integration efficiency with low non-productive gene destruction may be of particular importance for 'repeat approaches', i.e. if one aims for re-application of CRISPR/Cas modules to previously treated cells to increase integration efficiency. Because gene inactivation alters the guide RNA target sequence, only unaltered genes can be modified by the original CRISPR/Cas components while modified genes (without integration) are not susceptible to repeat approaches.

The exact determination of heterozygous and homozygous gene inactivation as well as non-specific and targeted integration events is of particular importance for the development and optimization of CRISPR/Cas derived therapies. The results presented herein demonstrate that gene inactivation occurred with much higher (>100fold) frequency than targeted integration. Even homozygous inactivation (both alleles affected) occurred with still 30-50fold higher frequency than targeted integration. Gene inactivation showed 'absolute' dependence on guide RNA specificity, while integration showed preferential integration at the target site but not to a degree that would be called 'specific' integration (2fold increased frequencies of target gene versus non-target gene integration). Because of that, currently available CRISPR/Cas derived gene editing technologies have to be evaluated specifically for applications that require defined integration events.

### Cited Documents:

Bauer, D. E., et al., 2015, Journal of visualized experiments : JoVE (95).
Brooks, S. C., et al., 1973, The Journal of biological chemistry 248(17):6251-3.
Carroll, D., 2011, Genetics 188(4):773-82.
Cho, S. W., et al., 2013, Nature biotechnology 31(3):230-2.
Cho, S. W., et al., 2014, Genome research 24(1):132-41.
Christian, M., et al., 2010, Genetics 186(2):757-61.
Cong, L., et al., 2013, Science 339(6121):819-23.
Cox, D. B., et al., 2015, Nature medicine 21(2):121-31.
Doench, J. G., et al., 2014, Nature biotechnology 32(12):1262-7.
Fu, Y., et al., 2013, Nature biotechnology 31(9):822-6.
Fu, Y., et al., 2014, Nature biotechnology 32(3):279-84.
Gaj, T., et al., 2013, Trends in biotechnology 31(7):397-405.
Gori, J. L., et al., 2015, Human gene therapy 26(7):443-51.
Gutschner T, et al., Cell Reports 14 (2016) 1555-1566.
Haeussler, M., et al., 2016, Genome biology 17(1):148.
Hockemeyer, D., et al., 2011, Nature biotechnology 29(8):731-4.
Holt, N., et al., 2010, Nature biotechnology 28(8):839-47.
Jinek, M., et al., 2012, Science 337(6096):816-21.
Jinek, M., et al., 2013, eLife 2:e00471.
Kleinstiver, B. P., et al. Nature 529 (2016) 490-495.
Li, H., et al. 2011, Nature 475(7355):217-21.
Liao, H. K., et al., 2015, Nature Communications 6.
Ma Y, et al., RNA Biol. 13 (2016) 605-12.
Makarova, K. S., et al., 2011, Nature reviews. Microbiology 9(6):467-77.
Mali, P., et al. 2013, Nature biotechnology 31(9):833-8.
Mali, P., et al. 2013, Science 339(6121):823-6.
Maruyama, T., et al., 2015, Nature biotechnology 33(5):538-42.
Mayer, K., et al. 2015, International J. of molecular sciences 16(11):27497-507.
McMahon, M. A., et al., 2012, Nature methods 9(1):28-31.
Miller, J. C., et al., 2007, Nature biotechnology 25(7):778-785.
Miller, J. C., et al., 2011, Nature biotechnology 29(2):143-8.
Muller, M., et al., 2016, Journal Am. Society of Gene Therapy 24(3):636-44.
Pattanayak, V., et al., 2013, Nature biotechnology 31(9):839-43.
Perez, E. E., et al., 2008, Nature biotechnology 26(7):808-16.
Pinder, J., et al., Nucl. Acids Res. 43 (2015) 9379-9392.
Ran, F. A., et al., 2013, Nature protocols 8(11):2281-308.
Sander, J. D., et al., 2011, Nature methods 8(1):67-U94.
Sanjana, N. E., et al., 2012, Nature protocols 7(1):171-92.
Shalem, O., et al., 2014, Science 343(6166):84-7.
Song, J., et al., Nature Comm. 2016-01-01.
Stahl, S., et al. 2015, Proc. Natl. Acad. Sci USA 112(34):10732-7.
Tebas, P., et al., 2014, The New England Journal of medicine 370(10):901-10.
Urnov, F. D., et al., 2010, Genetics 11(9):636-46.
Vara, J. A., et al., 1986, Nucleic acids research 14(11):4617-24.
Weidle, U.H., et al., Cancer Gen. Prot. 11 (2014) 25-38.
Wood, A. J., et al., 2011, Science 333(6040):307-307.
Yin, H., et al., 2014, Nature biotechnology 32(6):551-3.
Zhang, F., et al., 2011, Nature biotechnology 29(2):149-53.
Zhang, G., et al., 1996, Biochemical and biophysical research communications 227(3):707-11.
Zhang, X. H., et al., 2015, Nucleic acids 4:e264.

### Description of the Figures

- **Figure 1**: Composition of gRNAs: Sequence, size and exact genomic loci of DPH1 (A) and DPH2 (B) targeting gRNAs; exact chromosomal position is indicated in brackets; LHR: left homologous region and RHR: right homologous region of the integration cassette on the donor plasmid. Corresponding mRNA sequences are RefSeq: NM_001383 (DPH1) and RefSeq: NM_001039589, NM_001384 (DPH2).

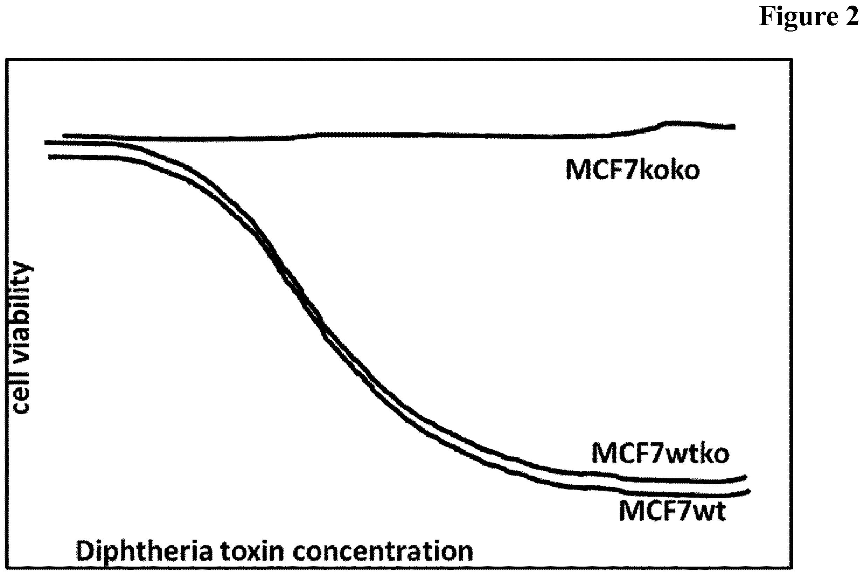

- **Figure 2**: MCF7 cells and MCF7 containing heterozygous (wt/k.o.) or homozygous (k.o./k.o.) inactivated DPH1 genes were exposed to different concentrations of diphtheria toxin for 48 hrs. Cell viability was assessed by viability assays as previously described (Mayer, et al. 2015; Stahl, et al. 2015).
- **Figure 3**: MCF7 cells were transfected with plasmids that encode CRISPR/Cas9 modules for inactivation of the Diphtheria toxin (DT) sensitivity gene DPH1, as well as for integration of the PAC expression cassette that confers Puromycin (PM) resistance. (A&B) 48hrs. after transfection, cells were exposed to DT at concentrations that are lethal for cells containing DPH1 functionality. This toxin selection generates survivor colonies (upper colony in A) in which all DPH1 gene copies are inactivated. Colonies that retain a functional DPH1 gene become killed by the toxin (colony fragments in A). Colonies can be visualized by staining and their number be quantified as colony counts depicted in (B). Note that DT-resistant colonies emerge only upon treating cells with DPH1 gene specific guide RNA without any nonspecific background in cells exposed to control guides. (C) 48 hrs. after transfection, cells were exposed to PM at concentrations that are lethal for cells that do not carry the PAC expression cassette. In consequence, PM selection generates survivor colonies that carry at least one PAC expression cassette. Colonies can be visualized by staining and their number be quantified as colony counts depicted in (C). Note that PM-resistant colonies emerge at higher numbers upon treating cells with guide RNA for specific integration into the DPH1 gene, but also show a background in cells exposed to control guides, indicating nonspecific integration events.
- **Figure 4**: MCF7wt cells, MCF7wtko cells with one DPH1 wild-type allele and one inactivated allele, and MCF7koko cells with both DPH1 genes inactivated were subjected to HRM PCR. The PCR fragment subjected to HRM spans the CRISPR/Cas target region in the DPH1 gene. Note that cells carrying a modification in the DPH1 gene can be differentiated from wild-type cells by differences in their melting curves, indicating the presence of altered alleles. Homozygous and heterozygous modifications can be differentiated from unmodified wild-type cells. The method does not differentiate between cells that are modified on only one allele (heterozygous, toxin sensitive) and cells that have both alleles inactivated (homozygous, toxin resistant). Toxin-sensitive cells have biphasic melting curves indicative for cells with one wild-type allele (conferred toxin sensitivity) and one mutated allele (deviant melting point).
- **Figure 5**: MCF7 cells transfected with DPH1 specific CRISPR/Cas modules were subjected to DT selection or to HRM-PCR followed by DT selection. (A) DT selection generates resistant colonies only with matching DPH1 guide RNA, no colonies emerge in untreated cells or in cells that received scrambled guide RNA. (B) HRM-PCR revealed the frequency of cells that become modified in their DPH1 genes on one or both alleles. Subsequent DT-sensitivity assays showed that the frequency of mono-allelic hits (toxin sensitive and HRM-positive) is twice as high as inactivation of both alleles (HRM-positive & toxin resistant).
- **Figure 6**: MCF7 cells transfected with DPH1 specific CRISPR/Cas modules were subjected to PM selection and/or to DT-selection. Transfection control shows neither DTr nor PMr colonies. (A) PM selection generates resistant colonies at 2-fold higher frequency with DPH1 guide RNA, compared to scrambled guide RNA. No colonies emerge in untreated cells. (B) Combination of PM selection and DT selection quantifies the frequency at which the PAC-cassette becomes integrated in cells whose DPH1 genes are completely inactivated. DPH1 guide RNA generates clones with PM-DT double resistances. Scrambled guide RNA generates only PM but not DT-resistant clones. (C) Comparison of the frequency of DT-resistant (both DPH1 genes inactivated) colonies and of PM-resistances (PAC integration at the DPH1 gene or at another site). (D) Shown are mean values + SEM (n=4, ^{∗∗∗}p<0.001). PM selection generates resistant colonies at 2-fold higher frequency with DPH1 gRNA, compared to scRNA. Combining PM selection and DT selection reveals the frequency at which the PAC-cassette becomes integrated in cells with both DPH1 alleles inactivated. DPH1 gRNA generates clones with PM-DT double resistances. ScRNA generates only PMr but not DTr colonies. (E) Shown are mean values + SEM (n=4, ^{∗∗∗}p<0.001). Comparison of the frequency of DTr (both DPH1 genes inactivated) colonies and PMr colonies (PAC integration at DPH1 or at another site). (F) Shown are mean values + SEM (n=4, ^{∗∗∗}p<0.001). MCF7 cells transfected with DPH2 specific gRNA were subjected to PM and/or to DT-selection.

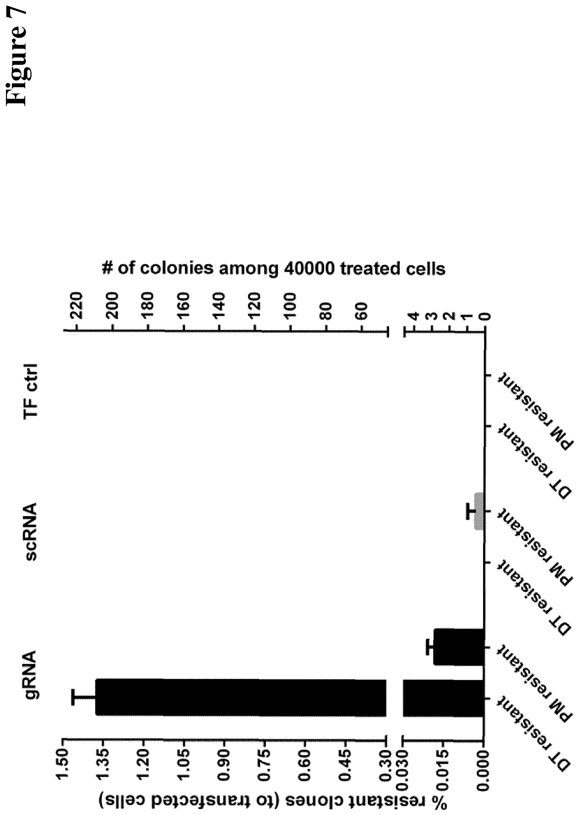

- **Figure 7**: MCF7 cells transfected with DPH2 specific CRISPR/Cas modules were subjected to PM selection and/or to DT-selection. In the same manner as observed for DPH1, frequencies of DT-resistant colonies are much higher than those of PM -resistant colonies. Thus, inactivation of both alleles is significantly more efficient than integration of the PAC cassette.
- **Figure 8**: Optimizing gene editing: influence of gRNA length and editing enzymes on efficacy and specificity.
(A) MCF7 cells transfected with DPH1 specific CRISPR/Cas modules are subjected to PM and DT selection with guide RNAs (gRNAs) of different length. The readouts enable the determination of absolute gene inactivation and cassette integration frequencies, as well as the determination of guide-RNA independent nonspecific integration events.
(B) The normalized ratio between frequencies of DT resistances and PM resistances identifies conditions at which desired cassette integration occurs with reduced events of non-integration-associated gene inactivation. Note also that maximum efficiency of destructive gene editing is achieved with 20mers while maximum integration efficiency is observed with 16-18mers.
(C) Maximum efficiency of productive integration at the target gene (cells that are resistant to PM as well as DT) is observed with 18mer guides.
(D) Shown are mean values + SEM (n=4, ΛΛ/ΦΦ/ΨΨP<0.01, ΛΛΛ/ΦΦΦ/ΨΨΨP<0.001). MCF-7 cells transfected with DPH1-specific Cas9 modules are subjected to PM and DT selection with gRNA of different length. gRNA length affects gene inactivation and integration frequencies. All scRNA/gRNA values were compared to the maximum value of the respective selection group.
(E) Shown are mean values + SEM (n=4, ΛΛ/ΦΦ/ΨΨP<0.01, ΛΛΛ/ΦΦΦ/ΨΨΨP<0.001). MCF-7 cells transfected with DPH1-specific Cas9 modules are subjected to PM and DT selection with different enzymes. Total number of DTr, PMr or DTr+PMr colonies in DPH1 editing approaches with 20mer gRNA (CRISPR/Cas9) or designed ZFN.
(F) Shown are mean values + SEM (n=4, ΛΛ/ΦΦ/ΨΨP<0.01, ΛΛΛ/ΦΦΦ/ΨΨΨP<0.001). MCF-7 cells transfected with DPH1-specific Cas9 modules are subjected to PM and DT selection with different enzymes. Ratio of total integration events to total inactivation events (PMr/DTr) and ratio of site-specific integration events/total target gene inactivation events (DTr+PMr)/DTr).
- **Figure 9**: Frequencies of specific and non-specific CRISPR/Cas mediated gene editing events observed with a 20mer guide RNA targeting the human DPH1 gene.
- **Figure 10**: Influence of DNA-repair modulating agents on gene editing. MCF-7 cells were transfected with plasmids encoding 20mer gRNA, SpCas9 and PAC. HR-modulating agent RS-1 (8µM) and NHEJ-modulating SCR7 (1 µM) were added either 4hrs before or 18hrs after transfection. DT and/or PM selection started 72hrs after transfection. Shown is the percentage of PMr colonies (integration) relative to DTr colonies (cleavage). Shown are mean values +SEM (n=4). Φ. Ap=0.05, ΦΦ, AAp=0.01, ΦΦΦΛΛΛp=0.001 versus untreated (no additional agent) control.

The following examples, figures and sequences are provided to aid the understanding of the present invention, the true scope of which is set forth in the appended claims.

### Examples

### Examnle 1

### Cultivation of MCF7 cells and transfection of plasmids encoding gene-editing entities

MCF7 cells (Brooks, et al. 1973) were originally obtained from ATCC (subsequently propagated in an in-house cell bank) and maintained in RPMI 1641 medium supplemented with 10% FCS, 2mM L-Glutamine and penicillin/streptomycin at 37°C and 85% humidity. For transfection of plasmids harboring gene-editing modules, 3,000,000 cells were seeded in a 10cm diameter culture dish and cultivated at 37°C and humidified 5% CO2. 24h after seeding, cells were transfected with 20µg total DNA using JetPEI (Polyplus) according to the manufacturer's protocol but with an N/P ratio of 6:1. Transfection efficiency was determined after 24h by flow cytometry (FACS Calibur, BD biosciences) of cells that were transfected with a modified eGFP expression plasmid (Zhang, et al. 1996).

Plasmids encoding CRISPR/Cas9 gene-editing entities (i.e. knock-out and integration systems) against Dphl (gRNA target sequence: CAGGGCGGCCGAGACGGCCC (SEQ ID NO: 01) derived from RefSeq: NM_001383) and Dph2 (gRNA target sequence: GATGTTTAGCAGCCCTGCCG (SEQ ID NO: 02) derived from RefSeq: NM_001039589, NM_001384), and scrambled control (gRNA sequence: GCACTACCAGAGCTAACTCA (SEQ ID NO: 03)) were obtained from OriGene. This system comprises one plasmid expressing a ~100nt gRNA under control of a U6 promoter as well as Cas9 nuclease under control of a CMV promoter and a Donor plasmid with a promoterless GFP/PM expression cassette flanked by homologous arms to the target gene (Dphl or Dph2). Additional Dphl gRNA sequence variants of different sizes (OriGene) were the

| | | |
|---|---|---|
| 14mer | GGCCGAGACGGCCC; | |
| 16mer | GCGGCCGAGACGGCCC; | |
| 18mer | GGGCGGCCGAGACGGCCC, | |
| 20mer | CAGGGCGGCCGAGACGGCCC; | |
| 22mer | AGCAGGGCGGCCGAGACGGCCC; | |
| 24mer | GGAGCAGGGCGGCCGAGACGGCCC; | and |
| 26mer | GCGGAGCAGGGCGGCCGAGACGGCCC | |

(see Figure 1A).

### Example 2

### Identification and quantification of CRISPR/Cas9 mediated homozygous DPH1 and DPH2 gene knockouts

MCF7 cells which have all chromosomal copies of DPH1 or DPH2 inactivated are resistant to diphtheria toxin (Stahl, et al. 2015). Thus, occurrence and frequency of toxin resistant cells/colonies upon CRISPR/Cas inflicted gene inactivation provides a measure for efficiency of inactivation of all gene copies. Therefore, MCF/ cells were transfected as described in Example 1 with (i) a GFP expression plasmid as transfection control, (ii) the CRISPR/Cas9 Dphl or Dph2 knock-out/integration system and (iii) knock-out/integration entities containing a scrambled gRNA to determine Cas9 independent integration frequencies. After determination of transfection efficiency, cells were seeded in 6-well plates. For quantification of homozygous knock-out events by DT 20000 cells were seeded and for quantification of integration events by PM or both events 40000 cells were seeded. RPMI medium was exchanged by RPMI medium containing DT or PM or both toxins 3 days after cell seeding. Exchange of medium was repeated every 2-3 days until all dead cells were removed from the plates and actively growing colonies could be detected via microscopic inspection. Thereafter (between day 12 and day 14 after start of toxin exposure), cells were washed 3 times with PBS and subsequently stained with ice cold methylene blue (0.2 % in 50 % EtOH). Methylene blue solution was subsequently removed and the plates were carefully washed under running water. Finally, cell colonies were counted under the microscope with a 5mm grid foil. Results of these analyses are summarized in the following Table.

**Table: Colony Counts & Phenotype Frequencies of transfected MCF-7 cells 'TF eff.': Transfection efficiency was determined by FACS analyses, monitoring frequencies of fluorescent cells upon transfection of MCF7 with GFP-reporter plasmids. Listed are relative numbers of GFP positive cells among all cells in %. The average transfection efficiency among all assays was between 30 -40%. 'HRM': High resolution melting point PCR positive cells are defined as those displaying an unambiguously divergent (biphasic) melting curve pattern in comparison to wildtype cells. 'K.O.' indicates the frequency of cells which carry no functional DPH1 or DPH2 gene copy and are hence resistant to DT. 'Int.' indicates the frequency of cells which harbor the PAC expression cassette and are hence resistant to PM. 'A-D' indicates individual samples of independent (quadruplicate) experiments.**

| **DPH1 HRM-PCR** | | **TF eff. [%]** | | | | | | |
|---|---|---|---|---|---|---|---|---|
| | | 37.8 | | | | | | |

| | | **# single cells** | | **# HRM⁺ cells** | | **#DT resistant** | | |
|---|---|---|---|---|---|---|---|---|
| | | 92 | | 6 | | 2 | | |
| | | | | | | | | |

| **DPH1 colony assay (K.O. vs. Int.)** | **TF eff. [%]** | | | | | | | |
|---|---|---|---|---|---|---|---|---|
| | 37.8 | | | | | | | |

| | **DT resistant** | | | | **PM resistant** | | | |
|---|---|---|---|---|---|---|---|---|
| | **# seeded cells** | | **# colonies** | | **# seeded cells** | | **# colonies** | |
| CRISPR Cas9 scRNA A | 40000 | | 0 | | 40000 | | 17 | |
| CRISPR Cas9 scRNA B | 40000 | | 0 | | 40000 | | 13 | |
| CRISPR Cas9 scRNA C | 40000 | | 0 | | 40000 | | 9 | |
| CRISPR Cas9 scRNA D | 40000 | | 0 | | 40000 | | 10 | |
| CRISPR Cas9 scRNA E | 20000 | | 0 | | 20000 | | 11 | |
| CRISPR Cas9 scRNA F | 20000 | | 0 | | 20000 | | 7 | |
| CRISPR Cas9 scRNA G | 20000 | | 0 | | 20000 | | 10 | |
| CRISPR Cas9 scRNA H | 20000 | | 0 | | 20000 | | 9 | |
| CRISPR Cas9 scRNA I | 10000 | | 0 | | 10000 | | 3 | |
| CRISPR Cas9 scRNA J | 10000 | | 0 | | 10000 | | 2 | |
| CRISPR Cas9 scRNA K | 10000 | | 0 | | 10000 | | 3 | |
| CRISPR Cas9 scRNA L | 10000 | | 0 | | 10000 | | 4 | |
| CRISPR Cas9 Dphl gRNA A | 40000 | | 995 | | 40000 | | 29 | |
| CRISPR Cas9 Dphl gRNA B | 40000 | | 942 | | 40000 | | 12 | |
| CRISPR Cas9 Dphl gRNA C | 40000 | | 899 | | 40000 | | 30 | |
| CRISPR Cas9 Dphl gRNA D | 40000 | | 950 | | 40000 | | 24 | |
| CRISPR Cas9 Dphl gRNA E | 20000 | | 364 | | 20000 | | 18 | |
| CRISPR Cas9 Dphl gRNA F | 20000 | | 545 | | 20000 | | 13 | |
| CRISPR Cas9 Dphl gRNA G | 20000 | | 543 | | 20000 | | 13 | |
| CRISPR Cas9 Dphl gRNA H | 20000 | | 435 | | 20000 | | 6 | |
| CRISPR Cas9 Dphl gRNA I | 10000 | | 201 | | 10000 | | 7 | |
| CRISPR Cas9 Dphl gRNA J | 10000 | | 224 | | 10000 | | 10 | |
| CRISPR Cas9 Dphl gRNA K | 10000 | | 219 | | 10000 | | 10 | |

| **DPH2 colony assay (K.O. vs. Int.)** | **TF eff. [%]** | | | | | | | |
|---|---|---|---|---|---|---|---|---|
| | 38 | | | | | | | |

| | **DT resistant** | | | | **PM resistant** | | | |
|---|---|---|---|---|---|---|---|---|
| | **# seeded cells** | | **# colonies** | | **# seeded cells** | | | **# colonies** |
| scRNA A | 40000 | | 0 | | 40000 | | | 2 |
| scRNA B | 40000 | | 0 | | 40000 | | | 0 |
| scRNA C | 40000 | | 0 | | 40000 | | | 0 |
| scRNA D | 40000 | | 0 | | 40000 | | | 0 |
| Dph2 CRISPR Cas9 gRNA A | 40000 | | 162 | | 40000 | | | 0 |
| Dph2 CRISPR Cas9 gRNA B | 40000 | | 227 | | 40000 | | | 0 |
| Dph2 CRISPR Cas9 gRNA C | 40000 | | 227 | | 40000 | | | 1 |
| Dph2 CRISPR Cas9 gRNA D | 40000 | | 215 | | 40000 | | | 3 |
| | | | | | | | | |

| **DPH1 col. assay (integr. vs. integr.+k.o.)** | | **TF eff. [%]** | | | | | | |
|---|---|---|---|---|---|---|---|---|
| | | 66.6^{∗} | | | | | | |

| | | **PM resistant** | | | | **PM+DT resistant** | | |
|---|---|---|---|---|---|---|---|---|
| | | # **seeded cells** | | # **colonies** | | # **seeded cells** | # **colonies** | |
| scRNA A | | 40000 | | 10 | | 40000 | 0 | |
| scRNA B | | 40000 | | 9 | | 40000 | 0 | |
| scRNA C | | 40000 | | 10 | | 40000 | 0 | |
| scRNA D | | 40000 | | 16 | | 40000 | 0 | |
| Dphl CRISPR Cas9 gRNA A | | 40000 | | 25 | | 40000 | 15 | |
| Dphl CRISPR Cas9 gRNA B | | 40000 | | 22 | | 40000 | 13 | |
| Dphl CRISPR Cas9 gRNA C | | 40000 | | 25 | | 40000 | 11 | |
| Dphl CRISPR Cas9 gRNA D | | 40000 | | 24 | | 40000 | 12 | |

| **DPH1 colony assay (gRNA length)** | **TF eff. [%]** | | | | | | | |
|---|---|---|---|---|---|---|---|---|
| | 31.2 | | | | | | | |

| | **DT resistant** | | **PM resistant** | | **PM + DT resistant** | | | |
|---|---|---|---|---|---|---|---|---|
| | **# seeded cells** | **# coloni es** | **# seeded cells** | **# coloni es** | **# seeded cells** | **# coloni es** | | |
| 20mer scRNA A | 20000 | 0 | 40000 | 7 | 40000 | 0 | | |
| 20mer scRNA B | 20000 | 0 | 40000 | 6 | 40000 | 0 | | |
| 20mer scRNA C | 20000 | 0 | 40000 | 9 | 40000 | 0 | | |
| 20mer scRNA D | 20000 | 0 | 40000 | 10 | 40000 | 0 | | |
| 14mer Dphl CRISPR Cas9 gRNA A | 20000 | 0 | 40000 | 6 | 40000 | 0 | | |
| 14mer Dphl CRISPR Cas9 gRNA B | 20000 | 0 | 40000 | 10 | 40000 | 0 | | |
| 14mer Dphl CRISPR Cas9 gRNA C | 20000 | 0 | 40000 | 8 | 40000 | 0 | | |
| 14mer Dphl CRISPR Cas9 gRNA D | 20000 | 0 | 40000 | 9 | 40000 | 0 | | |
| 16mer Dphl CRISPR Cas9 gRNA A | 20000 | 275 | 40000 | 21 | 40000 | 7 | | |
| 16mer Dphl CRISPR Cas9 gRNA B | 20000 | 298 | 40000 | 26 | 40000 | 9 | | |
| 16mer Dphl CRISPR Cas9 gRNA C | 20000 | 292 | 40000 | 25 | 40000 | 10 | | |
| 16mer Dphl CRISPR Cas9 gRNA D | 20000 | 271 | 40000 | 26 | 40000 | 9 | | |
| 18mer Dphl CRISPR Cas9 gRNA A | 20000 | 284 | 40000 | 19 | 40000 | 10 | | |
| 18mer Dphl CRISPR Cas9 gRNA B | 20000 | 299 | 40000 | 25 | 40000 | 12 | | |
| 18mer Dphl CRISPR Cas9 gRNA C | 20000 | 321 | 40000 | 27 | 40000 | 7 | | |
| 18mer Dphl CRISPR Cas9 gRNA D | 20000 | 324 | 40000 | 22 | 40000 | 11 | | |
| 20mer Dphl CRISPR Cas9 gRNA A | 20000 | 417 | 40000 | 16 | 40000 | 6 | | |
| 20mer Dphl CRISPR Cas9 gRNA B | 20000 | 406 | 40000 | 20 | 40000 | 5 | | |
| 20mer Dphl CRISPR Cas9 gRNA C | 20000 | 409 | 40000 | 16 | 40000 | 7 | | |
| 20mer Dphl CRISPR Cas9 gRNA D | 20000 | 404 | 40000 | 8 | 40000 | 7 | | |
| 22mer Dphl CRISPR Cas9 gRNA A | 20000 | 278 | 40000 | 14 | 40000 | 6 | | |
| 22mer Dphl CRISPR Cas9 gRNA B | 20000 | 292 | 40000 | 12 | 40000 | 5 | | |
| 22mer Dphl CRISPR Cas9 gRNA C | 20000 | 297 | 40000 | 11 | 40000 | 4 | | |
| 22mer Dphl CRISPR Cas9 gRNA D | 20000 | 267 | 40000 | 15 | 40000 | 5 | | |
| 24mer Dphl CRISPR Cas9 gRNA A | 20000 | 237 | 40000 | 13 | 40000 | 2 | | |
| 24mer Dphl CRISPR Cas9 gRNA B | 20000 | 252 | 40000 | 12 | 40000 | 4 | | |
| 24mer Dphl CRISPR Cas9 gRNA C | 20000 | 228 | 40000 | 11 | 40000 | 3 | | |
| 24mer Dph1 CRISPR | 20000 | 211 | 40000 | 15 | 40000 | 3 | | |
| Cas9 gRNA D | | | | | | | | |
| 26mer Dphl CRISPR Cas9 gRNA A | 20000 | 161 | 40000 | 12 | 40000 | 0 | | |
| 26mer Dphl CRISPR Cas9 gRNA B | 20000 | 155 | 40000 | 11 | 40000 | 1 | | |
| 26mer Dphl CRISPR Cas9 gRNA C | 20000 | 176 | 40000 | 12 | 40000 | 0 | | |
| 26mer Dphl CRISPR/Cas9 gRNA D | 20000 | 187 | 40000 | 9 | 40000 | 1 | | |

### Example 3

### Detection of CRISPR/Cas9 mediated heterozygous DPH1 and DPH2 gene knockouts

Cells which have only one allele of dph1 or dph2 modified are not toxin resistant. To identify and quantify those events, high resolution melting (HRM) PCR was applied in a similar manner as described by Stahl et al (Stahl, et al. 2015): 24h after transfection, single cells were deposited in 96-well plates by FACS (FACSAria, BD biosciences) and grown until confluency. Cells were washed with PBS and lysed by addition of 40µL cell lysis buffer (Roche) per well. After 15mins incubation at RT on a plate shaker (Titramax 1000, Heidolph) at 750 rpm, the cell lysate was 1:5 diluted with PCR grade H2O. 5µL cell lysate was mixed with a High resolution melting (HRM) master mix (Roche) and with primer spanning the gRNA target sequence. PCR and HRM were performed in an LC480 II (Roche) according the manufacturer's protocol. Clones with edited target genes were identified by their altered melting curve compared to MCF7-WT cells.

Cells with biphasic melting curves may still possess one wildtype allele or may have both alleles inactivated by cell viability assays. Therefore, such clones were expanded (without toxin or Puromycin selection) and subjected to cell viability analyses to discriminate between heterozygous (toxin sensitive) and homozygous (resistant) knockout cells. These assays were performed in flat bottom 96 well plates containing 10.000 cells at 37°C in humidified 5% CO2 conditions. 24hrs. after seeding, cells were exposed to toxin for 72h. Metabolic activity of surviving cells was assessed by a CellTiterGlo^{®} Luminescent Viability Assay (Promega) performed according to the manufacturer's specifications. Results of these analyses are summarized in the Table above.

### Example 4

### Detection of cassette integration events

CRISPR/Cas module for targeted integration contains a puromycin (PM) resistance gene expression cassette without promoter to avoid transient expression. Thus, detection of integration of recombinant sequences into the genome was detected by determining sensitivity of cells towards PM. A concentration of 500ng/µL PM (which quantitatively eliminates wildtype MCF7 cells) was applied to select MCF7 cells with stably integrated expression cassettes. PM - resistant colonies selected with the same procedure as described above for the DT selection and visualized and quantified (between 12 and 14 days after start of selection) in the same manner as described above for toxin resistant colonies. Number of PM resistant colonies obtained with non-target guide sequences (scrambled gRNA, Figure1) reflect the nonspecific integration background. Increase in colonies (above nonspecific background) obtained with target-gene gRNAs which are PM-resistant but toxin sensitive reflect integration into just one target allele, leaving the other dph1 or dph2 allele unaffected. PM resistant colonies that are also DT resistant have the expression cassette integrated into at least target allele and the other allele either inactivated or inactivated accompanied by yet another integration event. Results of these analyses are summarized in Table 1.

### Example 5

### The combination of Diphtheria toxin resistance assays and HRM-PCR differentiate between and quantify homozygous and heterozygous dph1 gene inactivation events

Diphtheria toxin (DT) ADP-ribosylates diphthamide on eukaryotic translation elongation factor 2 (eEF2) and thereby inactivates eEF2. This irreversibly stalls protein synthesis and kills cells. Diphthamide is a defined histidine which is placed by eEF2 by diphthamide synthesis genes, among them diphthamide biosynthesis 1 gene = DPH1. Complete inactivation of all DPH1 alleles in MCF7 cells (e.g. by gene editing) prevents the synthesis of the toxin target diphthamide. This renders cells resistant to Pseudomonas Exotoxin A (PE) and DT (Stahl et al.). In consequence, inactivation of all functional copies of DPH1 generates the phenotype 'DT-resistance' (Figure 3). The frequency of this phenotype can be detected in a rapid and robust manner by counting toxin resistant colonies as described in Examples 2-4 (Results in Table above and Figure3).

Because presence of just one remaining functional DPH1 allele is sufficient for toxin sensitivity (Figures 1 and 2), resistance phenotypes define specifically cells which harbor knockouts of all their DPH1alleeles. Cells which have only one allele modified can be identified by HRM-PCR assays performed directly on cultured cells (described in Example 3). Modification of the CRISPR/Cas target site alters the melting temperature of the dph1-gene derived PCR fragment compared to that of the wildtype fragment. This is reflected by a bi-phasic melting curve in HRM profiles (exemplarily shown in Figure 4). As CRISPR-Cas9 mediated gene-inactivation events are rarely identical on both alleles, many cells with complete gene inactivation will also show bi-phasic HRM profiles. These can be differentiated from mono-allelic gene alterations by their toxin resistant phenotype.

Thus, a combination of high throughput on-cell HRM-PCR and toxin selection (colony count) assays enables a quantification of heterozygous and homozygous DPH11 gene specific modification events.

### Example 6

### Puromycin resistance assays detect and differentiate the frequency of gene specific and non-specific integration events

Puromycin-N-acetyl-transferase (PAC) which is encoded by an integration cassette of the applied CRISPR/Cas9 plasmids inactivates PM and hence renders cells PM resistant (Vara, et al. 1986). Thus, integration of the PAC expression cassette can be detected and quantified by PM-resistance assays in the same manner as described above for DT-resistant colonies (applying PM instead of DT as selector, Figure 3). In contrast to DT resistance which results only from specific and homozygous target gene inactivation, PM resistance marks any integration event, independent from the position of integration. The frequencies of site specific vs nonspecific integration can be addressed by comparing number of PM-resistant cells exposed to target gene specific guide RNA's and of cells that were exposed to scrambled non-specific guides (see Figure 3).

### Example 7

### Comparison of CRISPR-Cas9 inflicted DPH1 gene inactivation and targeted integration events

To compare the frequencies of target-specific inactivation, integration and non-target integration, plasmids encoding DPH1 specific CRISPR-Cas9 modules (Example 1, Figure 1) were transfected into MCF7 cells. These were subsequently subjected to HRM-PCR as well as to colony count assays that detect DT- and PM resistances. The results of these assays are summarized in Figure 5, individual data sets are available in Table 1 above.

Figure 5A shows that complete inactivation of the DPH1 gene indicating functional loss of all DPH1 alleles occurred with a frequency of approx. 6% of all transfected cells (2.5% of all cells considering transfection efficiencies of 40%, Table 1). DPH1 gene inactivation showed absolute dependency on matching guide RNA sequence: scrambled guides did not generate any DT-resistant colony. A comparison of the frequencies of HRM 'hits' on the DPH1 gene with occurrence of DT resistant colonies is shown in Figure 5B. These analyses revealed that mono-allelic gene inactivation (toxin sensitive HRM-hit) occurred twice as frequent as inactivation of both alleles (DT-resistant cells).

Figure 6 shows frequencies of PM-resistant colonies and a comparison of the frequencies of DT-resistant colonies and PM-resistant colonies. These results indicated that simultaneous inactivation of both DPH1 alleles occurred with a 30-50 fold higher efficiency than integration of the PM-resistance mediating PAC expression cassette (Fig. 6A, without differentiation of position or zygosity of PAC integration). Compared to application of DPH1-specific guide RNA, scrambled guides generated (under otherwise identical conditions) 2 fold less PM-resistant colonies. This indicates that CRISPR/Cas9/DPH1-guide -mediated PAC-gene integration occurs with preference at the DPH1 gene, but not with absolute specificity. In accordance with preferential integration at the DPH1 target site, many PM-resistant colonies obtained with the DPH1 guide were toxin resistant (Fig. 6B). None of the PM-resistant colonies obtained with scrambled guides were DT resistant.

These results show that CRISPR/Cas9 mediated target gene inactivation (even simultaneously on both alleles) occurs highly specific and with much higher frequency than targeted integration (Figure 6C). Targeted integration occurs not only less frequent but is also less specific for the position defined by the guide RNA.

### Example 8

### Applicability and results of the assay/quantification approach for CRISPR/Cas9 gene editing can be transferred to other target genes

Are the results presented herein (incl. the observed large difference between targeted gene inactivation and integration) a general feature of CRISPR-Cas9 mediated gene editing or a particularity of the DPH1 gene? To address this question, the identical experimental approach was applied for CRISPR-Cas9 induced modification of the DPH2 gene. DPH2 is positioned on a different chromosome than DPH1, has a different sequence and encodes a different enzyme. But DPH2 is also essential for diphthamide synthesis and DPH2 deficiency renders cells resistant to DT in the same manner as DPH1 deficiency (Stahl et al.). Thereby, detection and quantification principles that are reported herein and have been developed to identify and differentiate DPH1 modulation can also be applied for the analysis of CRISPR/Cas9 mediated alterations of DPH2.

The results of DPH2 gene editing followed by assessment of DT- and PM-resistances are listed in Figure 7: In line with the observations on DPH1, homozygous DPH2 gene inactivation events were observed with approx. 40fold higher frequencies than integration of the PAC expression cassette. DPH2 inactivation was strictly dependent on presence of cognate guide RNA while cassette integration had site-preference but not absolute specificity for the target gene (comparing frequencies of DPH2 guide with scrambled guide RNA). The striking similarity of DPH1 and DPH2 gene editing results (even though both are different genes on different chromosomes) clearly shows that learnings and rules obtained with this assay system are transferable to other suitable genes and provides the reasonable basis that the herein reported method is generally applicable.

### Example 9

### Application of the method as reported herein: Comparison and optimization of gene manipulation modules

The outcomes of DPH1 and DPH2 gene editing experiments were highly comparable even though both are different genes on different chromosomes. It is therefore reasonable to assume that the 'rules' and learnings obtained with this system can be generalized to other genes. Thus, the method as reported herein can be applied for analyses that address general effects of the composition of gene modifying modules.

Therefore the method as reported herein has been applied for the comparison and optimization of gene manipulation modules. Figure 8 shows how gene inactivation as well as integration efficiency and specificity of CRISPR/Cas guide RNAs of different lengths can be assessed and compared regarding their efficiency and specificity of gene inactivation and/or integration. All applied guide RNAs targeted the same sequence stretch within DPH1 but varied in lengths from 14 to 26 bases (Figure 1 describes the guide RNAs in detail). Frequencies of DT-resistant colonies were recorded to reflect target gene specific homozygous gene inactivation. Simultaneously, numbers of PM resistant and DT-PM-double-resistant colonies were assessed to monitor cassette integration.

As expected, guide RNA length influences the efficiency of gene inactivation with 20mers conferring maximal DPH1 gene inactivation efficiency. Shortening the complementary stretch to 18 or 16 bases or extending it up to 26 bases retained significant specific gene inactivation functionality, albeit with decreased efficiency than the 20mer. Reducing guide RNA stretch to less than 16 bases (14mer) decreased DPH1 inactivating functionality to below detection levels.

Integration efficiency (assessed by counting PM-resistance events, compare and see Figure 5B) was also influenced by guide RNA lengths. Guides of less than 16mers (14mer) generated only few PM-resistant colonies, not exceeding scrambled control background levels. Targeted integration clearly above background was observed for 16mers, 18mers, 20mers, 22mers, 24mers and 26mers, reaching an optimum of insertion efficiency with 16-18mers. No efficiency gain was achieved with larger oligomeric nucleic acids, in fact larger size (incl. 20mers) reduced the specific insertion events significantly.

It has been found that the ratio between integration events (PM-r) and inactivation events (DT-r) can be calculated as a 'specificity indicator' to identify conditions at which specific integration occurs with the least gene inactivation events. Such conditions may be favored if one desired targeted integration without inflicting too much non-productive target gene damage.

Low values (e.g. few PM-r colonies in relation to DT-r colonies) reflect inefficient integration in relation to simultaneous occurring inactivation events. High values (more PM-r colonies and/or relatively decreased numbers of DT-r colonies) reflect more efficient integration at CRISPR/Cas affected target genes.

Figure 8B shows the calculated specificity factors in dependence on lengths of guide RNA. It has been observed that the highest 'insertion per inactivation' values are for 16-18mers and that there is a pronounced drop for guide RNAs containing 20 bases or more. This indicates that 20mers are quite efficient for targeted gene inactivation (in agreement with previous observations, describes guide RNAs in detail), yet shorter guides appear to be more efficient for specific integration. Shorter guides improve not only the integration efficiency (higher overall numbers of PM-r colonies), but also the ratio between productive and non-productive gene editing (reduction in DT-r colonies without insertion).

### Example 10

### Comparison of efficiency and specificity of different gene editing approaches

To compare gene inactivation and integration events, efficiency and specificity of different variants of the RNA-guided Cas9 as well as ZFN-mediated gene editing, the gRNA length and composition was kept constant (20mer, targeting DPH1). As a variable, three different editing enzymes were applied:
(i) 'SpCAS9' specifies the Cas9 nuclease from *Streptococcus pyogenes,* which can be considered as current standard application (Ran, Hsu et al. 2013, Fu, Sander et al. 2014);
(ii) SpCas9-HF1 is an engineered variant of SpCas9 with reduced unspecific DNA binding, reduced off-target activity, and hence with proposed higher fidelity & specificity (Kleinstiver, Pattanayak et al. 2016);
(iii) a ZFN-mediated gene editing module, which recognizes target sequences by designed zinc finger motifs, i.e. via protein-nucleic acid interactions as opposed to nucleic acid hybridization (Miller, Holmes et al. 2007, Urnov, Rebar et al. 2010).

In the same manner as described in the previous Example for gRNA length analyses, frequencies of DT-resistant colonies were recorded to reflect target gene specific and homozygous gene inactivation. PM resistant and DT-PM double resistant colonies were assessed to monitor cassette integration.

Comparing overall efficacies of gene inactivation and cassette integration, highest values for inactivation as well as integration were observed with CRISPR/SpCas9. For CRISPR/SpCas9-HF targeted gene inactivation events were lower, less than 20% of the number of DT-resistant colonies compared to CRISPR/SpCas9. The number of PM-resistant colonies (integration) and DT-PM double resistant colonies (cassette integration with targeted simultaneous gene inactivation) was also strongly reduced. Application of ZFN modules resulted in reduced numbers of DT-resistant colonies (with homozygous gene destruction) under otherwise identical conditions to less than 60% of the events observed with CRISPR/SpCas9. The efficiency of targeted gene inactivation was, thus, approx. 2-fold lower compared to the SpCAS9 and approx. 2-3 fold increased than that of the engineered SpCas9-HF1. The number of PM-resistant colonies (integration) did not significantly differ between CRISPR/SpCas9 and ZFN. Frequencies of DT-PM doubly resistant colonies (cassette integration with simultaneous gene inactivation) were somewhat (30%) reduced with ZFN compared to CRISPR/SpCas9.

The calculation of the ratio of DT-resistant (target gene inactivation) to DT+PM double-resistant (target site integration) colonies which takes overall efficiency out of the equation showed that CRISPR/SpCas9, as well as CRISPR/Cas9-HF, as well as the ZFN system generated the same level (approx. 4×10⁻³) of targeted integration events per one homozygous/complete gene inactivation event. Thus, while with the current editing systems the overall gene modification efficacies vary, 'specificities' of cassette integration above the background of target gene inactivation were determined to be not significantly different for all three evaluated approaches.

Thus, the method as reported herein can in consequence be applied to evaluate further editing systems or additives that support editing events to identify improved editing modules and/or conditions.

**Table: Colony counts and phenotype frequencies of MCF-7 cells transfected with different gene editing entities.**

| MCF-7 cells were transfected with plasmids encoding different genome editing systems (SpCas9, SpCas9, ZFN). The SpCas9 construct was applied as described above. SpCas9-HF includes the N497A/R661A/Q695A/Q926A substitutions according to Kleinstiver, Pattanayak et al. 2016. In parallel, gRNAs were replaced by scRNAs in parallel to address non-specific activity. DPH1-specific ZFN was obtained from Sigma Aldrich (CompoZr^{®}). The total amount of plasmid DNA (editing entity and donor) for transfection of the initial cell pool of 3×10⁶ cells was as described for the previous experiments. To quantify the transfection efficiency (TF eff. (%)), GFP-reporter plasmids were transfected aside. GFP-positive cells were counted 24hrs after transfection by FACS. Defined numbers of cells were seeded (#seeded cells) and treated with DT, PM, or DT+PM for 72hrs thereafter. | | | |
|---|---|---|---|
| **DPH1 assay editing entities** (TF eff. = 33%) | **# of DT resistant colonies** (40,000 seeded cells) | **# of PM resistant colonies** (80,000 seeded cells) | **# of DT+PM resistant colonies** (80,000 seeded cells) |
| SpCas9 scRNA | 0 | 4 | 0 |
| | 0 | 3 | 0 |
| | 0 | 5 | 0 |
| | 0 | 4 | 0 |
| SpCas9 Dphl gRNA | 476 | 14 | 5 |
| | 492 | 11 | 4 |
| | 468 | 9 | 4 |
| | 472 | 10 | 3 |
| SpCas9-HF scRNA | 0 | 2 | 0 |
| | 0 | 1 | 0 |
| | 0 | 0 | 0 |
| | 0 | 0 | 0 |
| SpCas9-HF Dphl gRNA | 60 | 9 | 1 |
| | 70 | 10 | 1 |
| | 66 | 8 | 0 |
| | 62 | 5 | 0 |
| ZFN-Dph1 | 286 | 12 | 4 |
| | 292 | 13 | 1 |
| | 276 | 10 | 2 |
| | 278 | 11 | 3 |

### Example 11

### Influence of DNA repair modulators on gene editing efficiency and specificity

The method as reported herein comprising colony assays to determine DT- and PM-resistant cells upon DPH gene editing can also be used to address the influence of compounds that modulate DNA repair mechanisms. Inhibitors of non-homologous end joining (NHEJ) processes, for example, have been described to modulate gene editing events (Ma, Y, et al. 2016). In the same manner, activators of homologous recombination (HR) may increase the efficiency of targeted cassette integration (Song, J, et al., 2016).

To evaluate and quantify the influence of DNA repair modulators on efficiency and specificity of gene editing, CRISPR/SpCAS9 and DPH1 targeting 20mer gRNA was combined with compounds that modulate DNA repair mechanisms. The inhibitor Scr7 was applied either 4hrs before transfection or 18hrs after transfection of the gene editing modules until 72hrs after transfection to inhibit DNA ligase IV. In the same manner, the RAD51 modulator (RS-1 (RAD51-stimulatory compound 1) was added to stimulate HR. Both compounds were applied at doses that had no effect on the growth or viability of MCF7 cells: 1 µM for Scr7 and 8 µM for RS-1, and 1 µM + 8 µM (Scr7+RS1) when combining both. The frequencies of DT-resistant, PM resistant and double-resistant colonies were subsequently recorded to reflect efficiency and specificity of target gene inactivation and cassette integration events under these different conditions.

It could be shown that modulation of NHEJ by addition of Scr7 4hrs before until 72hrs after transfection had no detectable influence on the number of DT-resistant colonies (reflecting frequency of target gene inactivation). It could also be shown that co-application of SCR7 and RS-1 4hrs before transfection, as well as application of Scr7 18hrs-72hrs after transfection lead to a small (yet significant) reduction of the number of DT resistant colonies compared to control transfections. Thus, it could be shown that DNA ligase IV mediated NHEJ may affect CRISPR/SpCAS9 inflicted target gene inactivation to some degree. Despite of its limited effect on the overall number of DT-resistant colonies, it could be shown that application of SCR7 4hrs before until 72hr after transfection significantly stimulated (approx. 2 fold, Table below) the frequency of PM-resistant colonies. This implies that SCR7 increases cassette integration events, confirming previous observations of enhanced productive gene editing upon administration of SCR7 (Ma Y et al 2016). Thus, these results provide proof that the method as reported herein can be used to determine such effects.

It could be shown that modulation of recombination by addition of the HR stimulator RS-1 4hrs before until 72hr after transfection increased the number of PM-resistant colonies approx. 2fold without affecting the number of colonies obtained with scrambled guides (Table below). It could also be shown that RS1 did not affect efficiency of cassette integration when it was applied 18 hrs-72hr after initiation of editing. Thus, these results provide proof that the method as reported herein can be used to determine such effects.

It could be shown that combining ligase IV (NHEJ) inhibition and HR stimulation in gene editing (by adding Scr7 as well as RS-1 4hrs before until 72hr after transfection) reduced the occurrence of DT resistant cells and further enhanced the frequency of PM resistant cells (Table below). It could be shown that this approach also resulted in the highest ratio of PM-resistant to DT-resistant colonies compared to all other approaches. Thus, these results provide proof that the method as reported herein can be used to determine such effects.

It can be concluded from these experiments that the method as reported herein can be used to determine the influence of compounds, compound combinations, timing of compound application as well as potential mechanisms that affect the outcome of gene editing approaches. Thus, the method as reported herein is therefore useful to identify compounds or conditions that enhance desired gene editing effects and reduce undesired 'side effects'.

**Table: Colony counts and phenotype frequencies of MCF-7 cells exposed to SCR7 and/or RS-1 during gene editing**

| MCF-7 cells were transfected with plasmids for SpCas9-mediated editing as described before. Equal numbers of cells were seeded (#seeded cells) and treated with DT or PM 72 hours after initiation of editing. a | | | | | | |
|---|---|---|---|---|---|---|
| (A) Scr7 (1 µM final concentration), RS-1 (8 µM final concentration) or Scr7+RS1 (1uM+8uM, respectively) was added 4hrs before transfection. | | | | | | |
| (B) Influence of the time of application (4hrs before vs 18 after transfection) RS1 and Scr7 on compound-modulated SpCas9-gRNA mediated gene editing. difference is significant, p<0.008.of | | | | | | |
| **A** | | | | | | |
| **compound** | **DPH1 assay editing entities** | **Mean DT resistant colonies (replicates)** (4×40.000 seeded cells) | **Mean PM resistant colonies (replicates)** (4×80.000 seeded cells) | **gRNA specific DT-r colonies** (gRNA-scRNA) | **gRNA specific PM-r colonies** (gRNA-scRNA) | **% PM resistant relative to DT resistant colonies** (PM-r / DT-r) |
| - | GFP control | 0 (0;0) | 0 (0;0) | 0 | 0 | - |
| - | SpCas9 Dphl gRNA | 200;5 (213;203;19 4;192) | 8.5 (6;11;8;9) | 200.5 | 4.5 | 4.2 % |
| | SpCas9-scRNA | 0 (0;0;0;0;) | 4 (2;5;4;5) | | | |
| RS-1 (8µM) | SpCas9 Dphl gRNA | 201;5 (196;202;21 0; 198) | 14 (11;13;15;17) | 201.5 | 10.2 | 6.9 %^{∗} |
| | SpCas9-scRNA | 0 (0;0;0;0;) | 3;8 (2;3;6;4) | | | |
| SCR7 (1µM) | SpCas9 Dphl gRNA | 205;3 (215;193;20 5;208) | 13;3 (11;11;16;15) | 205.3 | 9.5 | 6.5 %^{∗} |
| | SpCas9-scRNA | 0 (0;0;0;0;) | 3;5 (4;4;3;3) | | | |
| RS-1 + SCR7 (8 µM + 1 µM) | SpCas9 Dphl gRNA | 175 (183;175;17 7;165) | 14;3 (12;14;15;16) | 175 | 12 | 8.1 %^{∗} |
| | SpCas9-scRNA | 0 (0;0;0;0;) | 2.0 (2;2;2;2) | | | |

| **B** | | | | | | |
|---|---|---|---|---|---|---|
| **compound** | **time of compound addition** | **Mean DT resistant colonies (replicates)** | **% DT resistant colonies relative to untreated control** | **Mean PM resistant colonies (replicates)** | **% PM resistant colonies relative to untreated control** | **% PM resistant relative to DT resistant colonies** |
| - | 4hr before transfection | 200.5 (213;203;19 4;192) | 100 % | 8.5 (6;11;8;9) | 100 % | 4.2 % |
| | 18hr after transfection | 515 (522;513;50 7;518) | 100 % | 20 (20;24;17;1 9) | 100 % | 3.9 % |
| RS-1 (8 µM) | 4hr before transfection | 201.5 (196;202;21 0;198) | 100 % | 14 (11;13;15;1 7) | 165 %^{∗} | 6.9 %^{∗} |
| | 18hr after transfection | 512.5 (512;521;50 6;511) | 100 % | 15.3 (17;15;14;1 5) | 76% | 3.0 % |
| SCR7 (1 µM) | 4hr before transfection | 205.3 (215;193;20 5;208) | 102 % | 13.3 (11;11;16;1 5) | 156 %^{∗} | 6.5 %^{∗} |
| | 18hr after transfection | 488.8 (486;482;49 1;496) | 95 %^{∗} | 25 (26;25;27;2 2) | 125% | 5.1 % |
| RS-1 + SCR7 (8 + 1 µM) | 4hr before transfection | 175 (183;175;17 7;165) | 87 %^{∗} | 14.3 (12;14;15;1 6) | 168 %^{∗} | 8.1 %^{∗} |
| | 18hr after transfection | 488.3 (492;485;49 5;481) | 95 %^{∗} | 10;.3 (8;10;6;17) | 51 % | 2.1 % |

### Example 12

### Identification and quantification of ZFN-mediated DPH1 gene editing

MCF7 cells which have all chromosomal copies of DPH1 inactivated are DT resistant. Thus, occurrence and frequency of DTr colonies upon ZFN inflicted gene inactivation and/or cassette integration provides a measure for efficacy of inactivation of all gene copies. The ZFN recognition sequence (CAGGTGATGGCGGCGCTGGTCGTATCCGGGGCAGCGGAGCAG, cleavage site, SEQ ID NO: 10) are derived from NM_001383.3 (DPH1-wt) and were obtained from Sigma Aldrich. A PAC integration cassette for this position was obtained from OriGene. MCF7 cells were transfected as described above with (i) a GFP expression plasmid, (ii) the plasmid encoding DPH1-targeting ZFN and (iii) the DPH1-targeting PAC-integration cassette. After determination of transfection efficiency, cells were seeded in 6-well plates. For quantification of homozygous knock-out events (DTr) 20,000 cells were seeded, 40,000 cells for quantification of integration events (PMr) or double resistances. RPMI medium was exchanged to RPMI containing DT or PM or both 3 days after seeding. Medium was changed every 2-3 days. Between day 12 and day 14 after starting toxin exposure, cells were washed 3 times with PBS and stained with ice cold methylene blue (0.2% in 50% EtOH) followed by washing under running water and microscopic determination of counting colonies numbers with a 5mm grid foil.

### Example 13

### Quantification of the effects of HR- and NHEJ-modulators on CRISPR/Cas9 mediated editing

The RAD51-stimulatory compound 1 (RS-1) was applied to modulate homologous recombination (HR) during gene editing. RS-1 (Sigma Aldrich) was dissolved in DMSO to generate a stock solution of 10 mg/ml, which was diluted in RPMI medium just before application to cells. Viability (Promega CTG) assays identified a final concentration of 8 µM RS-1 as a dose that does not inflict growth inhibitory or toxic effects on MCF7 (viability: 1 µM-100%; 3.7 µM-100%; 11 µM-97%; 33 µM-61%).

The DNA ligase IV inhibitor SCR7 was applied to modulate non-homologous end joining (NHEJ) during gene editing. SCR7 (Sigma) was dissolved in DMSO to generate a stock solution of 10 mg/ml, which was diluted in RPMI medium just before application to cells. Viability (Promega CTG) assays identified a final concentration of 1 µM as a dose that does not inflict growth inhibitory or toxic effects on MCF7 (viability: 0.37 µM-100%; 1.1 µM-100%; 3.3 µM-97%; 10 µM-88%).

SCR7 (1 µM final conc.) or RS-1 (8 µM final conc.) or SCR7+RS-1 (1 µM + 8 µM final conc.) were added to MCF7 cells 4hrs before transfection of the gene editing modules in the 'early exposure' setting. For 'late exposure' SCR7 (8 µM final conc.) or RS-1 (1 µM final conc.) were added to MCF7 cells 18hrs after transfection. In both settings, cells were exposed to the modulators until 96hr after transfection, i.e. 'early exposure' consisted of a treatment for a total of 100hrs, 'late exposure' for a total of 78hrs.

The system upon which to determine the effects of DNA repair modulators consisted of the CRISPR/SpCas9 modules with DPH1 20mer gRNA, transfected into MCF7 cells and subjected to subsequent DT and PM selection as described in the previous Examples. Frequencies of DTr, PMr, and double-resistant colonies were recorded to reflect gene inactivation and cassette integration events.

### Example 14

### Statistics

Unpaired, two-tailed Student's t-tests were performed for single comparisons between two treatments. Multiple comparisons were statistically analyzed by a one-way ANOVA followed by a Tukey's honestly different significance (HDS) post hoc test. A significant difference was defined by a p-value of < 0.05. The level of significance determined by student's t-test is indicated in graphs by one, two or three asterisks corresponding to p < 0.05, p < 0.01 and p < 0.001. Likewise, the level of significance determined by Tukey's HDS test is indicated by Λ, Φ or Ψ.

## Claims

1. Method for determining the efficiency and specificity of the introduction of a nucleic acid into the genome of a mammalian cell, whereby the mammalian cell comprises one or two transcriptionally active alleles of a DPH1, DPH2, DPH4 and/or DPH5 gene, comprising the steps of
- transfecting the mammalian cell with one or more plasmids comprising the nucleic acid to be introduced, a nucleic acid conferring resistance to a selection marker, and the elements required for gene editing of said DPH gene,
- cultivating the transfected cell in the absence of selection pressure,
- depositing the cells of the transfected multitude of cells as single cells,
- cultivating a first number of single deposited cells in the presence of a DPH gene transcription sensitive toxin, and cultivating a second number of single deposited cells in the presence of the corresponding selection marker,
- determining the number of toxin resistant colonies and the number of antibiotic resistant colonies, wherein the ratio between integration events represented by the number of antibiotic resistant colonies and inactivation events represented by the number of toxin resistant colonies reflects the specificity of the method.

2. The method according to claim 1, wherein the DPH gene transcription sensitive toxin is selected from the group consisting of pseudomonas exotoxin and diphtheria toxin.

3. The method according to any one of claims 1 to 2, wherein the introduction of the nucleic acid is a homozygous nucleic acid introduction into the genome of the mammalian cell if the transfected cell is viable in the presence of the toxin.

4. The method according to any one of claims 1 to 3, wherein the introduction of the nucleic acid is a heterozygous or non-site specific nucleic acid introduction into the genome of the mammalian cell if the transfected cell is not viable in the presence of the toxin but viable in the presence of the selectable marker.

5. The method according to any one of claims 1 to 4, wherein DPH gene inactivation and nucleic acid integration events are quantified by a combination of toxin and selectable marker selection, and optionally high-resolution melting (HRM) PCR.

6. The method according to any one of claims 1 to 5, wherein the inactivation of one allele of a target gene is detected by HRM-PCR by the presence of a bi-phasic melting curve.

7. The method according to claim 6, wherein the HRM-PCR is performed directly on cultured cells.

8. The method according to any one of claims 1 to 7, wherein the frequency of the inactivation of all alleles of a target gene by CRISPR/Cas9 is detected by counting toxin resistant colonies in combination with a bi-phasic melting curve determined by HRM-PCR.

## Patentansprüche

1. Verfahren zum Bestimmen der Wirksamkeit und Spezifität der Einbringung einer Nukleinsäure in das Genom einer Säugetierzelle, wobei die Säugetierzelle ein oder zwei transkriptionell aktive Allele eines DPH1-, DPH2-, DPH4- und/oder DPH5-Gens umfasst, umfassend die folgenden Schritte
- Transfizieren der Säugetierzelle mit einem oder mehreren Plasmiden, umfassend die einzubringende Nukleinsäure, eine Nukleinsäure, die Resistenz gegen einen Selektionsmarker verleiht, und die Elemente, die für Geneditierung des DPH-Gens erforderlich sind,
- Kultivieren der transfizierten Zelle in der Abwesenheit von Selektionsdruck,
- Ablagern der Zellen der transfizierten Vielzahl von Zellen als einzelne Zellen,
- Kultivieren einer ersten Anzahl von einzelnen abgelagerten Zellen in der Gegenwart eines DPH-Gentranskription-sensitiven Toxins und Kultivieren einer zweiten Anzahl von einzelnen abgelagerten Zellen in der Gegenwart des entsprechenden Selektionsmarkers,
- Bestimmen der Anzahl an Toxin-resistenten Kolonien und der Anzahl an Antibiotika-resistenten Kolonien, wobei das Verhältnis zwischen Integrationsereignissen, dargestellt durch die Anzahl an Antibiotika-resistenten Kolonien, und Inaktivierungsereignissen, dargestellt durch die Anzahl an Toxin-resistenten Kolonien, die Spezifität des Verfahrens wiedergibt.

2. Verfahren nach Anspruch 1, wobei das DPH-Gentranskription-sensitive Toxin aus der Gruppe, bestehend aus Pseudomonas-Exotoxin und Diphtherietoxin, ausgewählt ist.

3. Verfahren nach einem der Ansprüche 1 bis 2, wobei die Einbringung der Nukleinsäure eine homozygote Nukleinsäureeinbringung in das Genom der Säugetierzelle ist, wenn die transfizierte Zelle in der Gegenwart des Toxins lebensfähig ist.

4. Verfahren nach einem der Ansprüche 1 bis 3, wobei die Einbringung der Nukleinsäure eine heterozygote oder nicht-ortsspezifische Nukleinsäureeinbringung in das Genom der Säugetierzelle ist, wenn die transfizierte Zelle in der Gegenwart des Toxins nicht lebensfähig ist, aber in der Gegenwart des selektierbaren Markers lebensfähig ist.

5. Verfahren nach einem der Ansprüche 1 bis 4, wobei DPH-Geninaktivierungs- und Nukleinsäureintegrationsereignisse durch eine Kombination der Auswahl aus Toxin und selektierbarem Marker und gegebenenfalls hochauflösende Schmelz-(HRM)-PCR quantifiziert werden.

6. Verfahren nach einem der Ansprüche 1 bis 5, wobei die Inaktivierung eines Allels eines Zielgens durch HRM-PCR durch die Gegenwart einer Zweiphasen-Schmelzkurve nachgewiesen wird.

7. Verfahren nach Anspruch 6, wobei die HRM-PCR direkt an kultivierten Zellen ausgeführt wird.

8. Verfahren nach einem der Ansprüche 1 bis 7, wobei die Häufigkeit der Inaktivierung aller Allele eines Zielgens durch CRISPR/Cas9 durch Zählen Toxin-resistenter Kolonien in Kombination mit einer Zweiphasen-Schmelzkurve, bestimmt durch HRM-PCR, nachgewiesen wird.

## Revendications

1. Procédé de détermination de l'efficacité et de la spécificité de l'introduction d'un acide nucléique dans le génome d'une cellule de mammifère, la cellule de mammifère comprenant ainsi un ou deux allèles transcriptionnellement actifs d'un gène DPH1, DPH2, DPH4 et/ou DPH5, comprenant les étapes de
- transfection de la cellule de mammifère avec un ou plusieurs plasmides comprenant l'acide nucléique à introduire, un acide nucléique conférant une résistance à un marqueur de sélection et les éléments requis pour l'édition génique dudit gène DPH,
- la culture de la cellule transfectée en l'absence d'une pression de sélection,
- le dépôt des cellules de la multitude de cellules transfectées en tant que cellules uniques,
- la culture d'un premier nombre de cellules déposées uniques en présence d'une toxine sensible à la transcription du gène DPH et la culture d'un second nombre de cellules déposées uniques en présence du marqueur de sélection correspondant,
- la détermination du nombre de colonies résistantes à la toxine et du nombre de colonies résistantes à l'antibiotique, le rapport entre les événements d'intégration représentés par le nombre de colonies résistantes à l'antibiotique et les événements d'inactivation représentés par le nombre de colonies résistantes à la toxine reflétant la spécificité du procédé.

2. Procédé selon la revendication 1, dans lequel la toxine sensible à la transcription du gène DPH est choisie dans le groupe constitué par l'exotoxine de pseudomonas et la toxine diphtérique.

3. Procédé selon l'une quelconque des revendications 1 à 2, dans lequel l'introduction de l'acide nucléique est une introduction d'acide nucléique homozygote dans le génome de la cellule de mammifère si la cellule transfectée est viable en présence de la toxine.

4. Procédé selon l'une quelconque des revendications 1 à 3, dans lequel l'introduction de l'acide nucléique est une introduction d'acide nucléique hétérozygote ou non spécifique à un site dans le génome de la cellule de mammifère si la cellule transfectée n'est pas viable en présence de la toxine mais viable en présence du marqueur sélectionnable.

5. Procédé selon l'une quelconque des revendications 1 à 4, dans lequel les événements d'inactivation du gène DPH et d'intégration d'acide nucléique sont quantifiés par une combinaison de toxine et de sélection de marqueur sélectionnable, et éventuellement par une PCR de fusion haute résolution (HRM).

6. Procédé selon l'une quelconque des revendications 1 à 5, dans lequel l'inactivation d'un allèle d'un gène cible est détectée par PCR HRM par la présence d'une courbe de fusion biphasique.

7. Procédé selon la revendication 6, dans lequel la PCR HRM est effectuée directement sur des cellules cultivées.

8. Procédé selon l'une quelconque des revendications 1 à 7, dans lequel la fréquence de l'inactivation de l'ensemble des allèles d'un gène cible par CRISPR/Cas9 est détectée en comptant les colonies résistantes à la toxine en combinaison avec une courbe de fusion biphasique déterminée par PCR HRM


### SEQUENCE LISTING

<110> F. Hoffmann-La Roche AG
<120> Method to analyze and optimize gene editing modules and delivery approaches
<130> P33874-WO
<150> EP16191511.1
   <151> 2016-09-29
<150> EP17156440.4
   <151> 2017-02-16
<160> 10
<170> PatentIn version 3.5
<210> 1
   <211> 20
   <212> DNA
   <213> Artificial Sequence
<220>
   <223> gRNA Dphl
<400> 1
   cagggcggcc gagacggccc 20
<210> 2
   <211> 20
   <212> DNA
   <213> Artificial Sequence
<220>
   <223> gRNA Dph2
<400> 2
   gatgtttagc agccctgccg 20
<210> 3
   <211> 20
   <212> DNA
   <213> Artificial Sequence
<220>
   <223> scrambled control gRNA
<400> 3
   gcactaccag agctaactca 20
<210> 4
   <211> 14
   <212> DNA
   <213> Artificial Sequence
<220>
   <223> 14mer gRNA Dphl
<400> 4
   ggccgagacg gccc 14
<210> 5
   <211> 16
   <212> DNA
   <213> Artificial Sequence
<220>
   <223> 16mer gRNA Dph1
<400> 5
   gcggccgaga cggccc 16
<210> 6
   <211> 18
   <212> DNA
   <213> Artificial Sequence
<220>
   <223> 18mer gRNA Dph1
<400> 6
   gggcggccga gacggccc 18
<210> 7
   <211> 22
   <212> DNA
   <213> Artificial Sequence
<220>
   <223> 22mer gRNA Dph1
<400> 7
   agcagggcgg ccgagacggc cc 22
<210> 8
   <211> 24
   <212> DNA
   <213> Artificial Sequence
<220>
   <223> 24mer gRNA Dph1
<400> 8
   ggagcagggc ggccgagacg gccc 24
<210> 9
   <211> 26
   <212> DNA
   <213> Artificial Sequence
<220>
   <223> 26mer gRNA Dph1
<400> 9
   gcggagcagg gcggccgaga cggccc 26
<210> 10
   <211> 42
   <212> DNA
   <213> Artificial Sequence
<220>
   <223> ZFN recognition sequence
<400> 10
   caggtgatgg cggcgctggt cgtatccggg gcagcggagc ag 42